# EUROPÄISCHE PATENTSCHRIFT

(11) **EP 3 199 633 B1**
(45) Veröffentlichungstag und Bekanntmachung des Hinweises auf die Patenterteilung: **14.11.2018**
(21) Anmeldenummer: 17151870.7
(22) Anmeldetag: 12.09.2008
(51) Int. Cl.: C12N 15/11, C12N 7/04, A61K 31/713, A61K 47/42, C07K 14/025

(54) **HERABREGULATION DER GENEXPRESSION MITTELS NUKLEINSÄURE-BELADENER VIRUS-ÄHNLICHER PARTIKEL**
DOWNREGULATION OF GENE EXPRESSION USING PARTICLES CHARGED WITH NUCLEIC ACID SIMILAR TO VIRUSES
RÉGULATION À LA BAISSE DE L'EXPRESSION GÉNIQUE À L'AIDE DE PARTICULES PSEUDO-VIRALES CHARGÉES D'ACIDE NUCLÉIQUE

(30) Priorität: 14.09.2007 EP 07018130
(43) Veröffentlichungstag der Anmeldung: 02.08.2017
(62) Teilanmeldung aus: 08802132.4
(73) Patentinhaber: Deutsches Primatenzentrum GmbH, 37077 Göttingen (DE)
(72) Erfinder: Lüke, Wolfgang, 48153 Münster (DE); Gruber, Jens, 37077 Göttingen (DE)
(74) Vertreter: Krauss, Jan

(56) Entgegenhaltungen:
- EP-A- 0 962 525
- WO-A-99/36545
- WO-A-2006/119096

### SEQUENCE LISTING

<110> Deutsches Primatenzentrum GmbH
<120> Herabregulation der Genexpression mittels Nukleinsaeure-beladener virusaehnlicher Partikel
<130> FB22839-A
<150> EP07018130.0
   <151> 2007-09-14
<160> 7
<170> PatentIn version 3.3
<210> 1
   <211> 1065
   <212> DNA
   <213> Artificial
<220>
   <223> Polyoma JC VP1 Gen
<220>
   <221> misc_feature
   <222> (1)..(1065)
<400> 1
   atggccccaa caaaaagaaa aggagaaagg aaggaccccg tgcaagttcc aaaacttctt      60
   ataagaggag gagtagaagt tctagaagtt aaaactgggg ttgactcaat tacagaggta     120
   gaatgctttt taactccaga aatgggtgac ccagatgagc atcttagggg ttttagtaag     180
   tcaatttcta tatcagatac atttgaaagt gactccccaa ataaggacat gcttccttgt     240
   tacagtgtgg ccagaattcc actacccaat ctaaatgagg atctaacctg tggaaatata     300
   ctaatgtggg aggctgtgac cttaaaaact gaggttttag gggtgacaac tttgatgaat      360
   gtgcactcta atggtcaagc aactcatgac aatggtgcag gaaagccagt gcagggcacc     420
   agctttcatt ttttttctgt tgggggggag gctttagaat tacagggggt ggtttttaac     480
   tacagaacaa agtacccaga tggaacaatt tttccaaaga atgcaacagt gcaatctcaa     540
   gtaatgaaca cagagcacaa ggcgtaccta gataagaaca aagcatatcc tgttgaatgt     600
   tgggttcctg atcccaccag aaatgaaaac acaagatatt ttgggacact aacaggagga     660
   gaaaatgttc ctccagttct tcatataaca aacactgcca caacagtgct gcttgatgaa     720
   tttggtgttg ggccactttg caaaggtgac aacttgtatt tgtcagctgt tgatgtttgt     780
   ggaatgttta ctaacagatc tggtacccag cagtggagag gactgtccag atattttaag     840
   gttcagctga gaaaaaggag ggttaaaaac ccctacccaa tttctttcct tcttactgat     900
   ttgattaaca gaaggacccc tagagttgat gggcagccta tgtatggtat ggatgctcag     960
   gtagaggagg ttagagtttt tgaggggaca gaggaacttc caggggaccc agacatgatg    1020
   agatatgttg acagatatgg acagttgcaa acaaagatgc tgtaa                    1065
<210> 2
   <211> 354
   <212> PRT
   <213> Artificial
<220>
   <223> Polyoma VP1
<220>
   <221> MISC_FEATURE
   <222> (1)..(354)
<400> 2
   Met Ala Pro Thr Lys Arg Lys Gly Glu Arg Lys Asp Pro Val Gln Val
   1               5                   10                  15
   Pro Lys Leu Leu Ile Arg Gly Gly Val Glu Val Leu Glu Val Lys Thr
               20                  25                  30
   Gly Val Asp Ser Ile Thr Glu Val Glu Cys Phe Leu Thr Pro Glu Met
           35                  40                  45
   Gly Asp Pro Asp Glu His Leu Arg Gly Phe Ser Lys Ser Ile Ser Ile
       50                  55                  60
   Ser Asp Thr Phe Glu Ser Asp Ser Pro Asn Lys Asp Met Leu Pro Cys
   65                  70                  75                  80
   Tyr Ser Val Ala Arg Ile Pro Leu Pro Asn Leu Asn Glu Asp Leu Thr
                   85                  90                  95
   Cys Gly Asn Ile Leu Met Trp Glu Ala Val Thr Leu Lys Thr Glu Val
               100                 105                 110
   Leu Gly Val Thr Thr Leu Met Asn Val His Ser Asn Gly Gln Ala Thr
           115                 120                 125
   His Asp Asn Gly Ala Gly Lys Pro Val Gln Gly Thr Ser Phe His Phe
       130                 135                 140
   Phe Ser Val Gly Gly Glu Ala Leu Glu Leu Gln Gly Val Val Phe Asn
   145                 150                 155                 160
   Tyr Arg Thr Lys Tyr Pro Asp Gly Thr Ile Phe Pro Lys Asn Ala Thr
                   165                 170                 175
   Val Gln Ser Gln Val Met Asn Thr Glu His Lys Ala Tyr Leu Asp Lys
   180 185 190
   Asn Lys Ala Tyr Pro Val Glu Cys Trp Val Pro Asp Pro Thr Arg Asn
   195 200 205
   Glu Asn Thr Arg Tyr Phe Gly Thr Leu Thr Gly Gly Glu Asn Val Pro
   210 215 220
   Pro Val Leu His Ile Thr Asn Thr Ala Thr Thr Val Leu Leu Asp Glu
   225 230 235 240
   Phe Gly Val Gly Pro Leu Cys Lys Gly Asp Asn Leu Tyr Leu Ser Ala
   245 250 255
   Val Asp Val Cys Gly Met Phe Thr Asn Arg Ser Gly Thr Gln Gln Trp
   260 265 270
   Arg Gly Leu Ser Arg Tyr Phe Lys Val Gln Leu Arg Lys Arg Arg Val
   275 280 285
   Lys Asn Pro Tyr Pro Ile Ser Phe Leu Leu Thr Asp Leu Ile Asn Arg
   290 295 300
   Arg Thr Pro Arg Val Asp Gly Gln Pro Met Tyr Gly Met Asp Ala Gln
   305 310 315 320
   Val Glu Glu Val Arg Val Phe Glu Gly Thr Glu Glu Leu Pro Gly Asp
   325 330 335
   Pro Asp Met Met Arg Tyr Val Asp Arg Tyr Gly Gln Leu Gln Thr Lys
   340 345 350
   Met Leu
<210> 3
   <211> 1089
   <212> DNA
   <213> Artificial
<220>
   <223> Polyoma VP1-Mut2
<220>
   <221> misc_feature
   <222> (1)..(1089)
<400> 3
   atggccccaa caaaaagaaa aggagaatgt ccaggggcag ctcccaaaaa accaaaggac 60
   cccgtgcaag ttccaaaact tcttataaga ggaggagtag aagttctaga agttaaaact 120
   ggggttgact caattacaga ggtagaatgc tttttaactc cagaaatggg tgacccagat 180
   gagcatctta ggggttttag taagtcaatt tctatatcag atacatttga aagtgactcc 240
   ccaaataagg acatgcttcc ttgttacagt gtggccagaa ttccactacc caatctaaat 300
   gaggatctaa cctgtggaaa tatactaatg tgggaggctg tgaccttaaa aactgaggtt 360
   ttaggggtga caactttgat gaatgtgcac tctaatggtc aagcaactca tgacaatggt 420
   gcaggaaagc cagtgcaggg caccagcttt catttttttt ctgttggggg ggaggcttta 480
   gaattacagg gggtggtttt taactacaga acaaagtacc cagatggaac aatttttcca 540
   aagaatgcaa cagtgcaatc tcaagtaatg aacacagagc acaaggcgta cctagataag 600
   aacaaagcat atcctgttga atgttgggtt cctgatccca ccagaaatga aaacacaaga 660
   tattttggga cactaacagg aggagaaaat gttcctccag ttcttcatat aacaaacact 720
   gccacaacag tgctgcttga tgaatttggt gttgggccac tttgcaaagg tgacaacttg 780
   tatttgtcag ctgttgatgt ttgtggaatg tttactaaca gatctggtac ccagcagtgg 840
   agaggactgt ccagatattt taaggttcag ctgagaaaaa ggagggttaa aaacccctac 900
   ccaatttctt tccttcttac tgatttgatt aacagaagga cccctagagt tgatgggcag 960
   cctatgtatg gtatggatgc tcaggtagag gaggttagag tttttgaggg gacagaggaa 1020
   cttccagggg acccagacat gatgagatat gttgacagat atggacagtt gcaaacaaag 1080
   atgctgtaa 1089
<210> 4
   <211> 362
   <212> PRT
   <213> Artificial
<220>
   <223> VP1-Mut2
<220>
   <221> MISC_FEATURE
   <222> (1)..(362)
<400> 4
   Met Ala Pro Thr Lys Arg Lys Gly Glu Cys Pro Gly Ala Ala Pro Lys
   1               5                   10                  15
   Lys Pro Lys Asp Pro Val Gln Val Pro Lys Leu Leu Ile Arg Gly Gly
               20                  25                  30
   Val Glu Val Leu Glu Val Lys Thr Gly Val Asp Ser Ile Thr Glu Val
           35                  40                  45
   Glu Cys Phe Leu Thr Pro Glu Met Gly Asp Pro Asp Glu His Leu Arg
       50                  55                  60
   Gly Phe Ser Lys Ser Ile Ser Ile Ser Asp Thr Phe Glu Ser Asp Ser
   65                  70                  75                  80
   Pro Asn Lys Asp Met Leu Pro Cys Tyr Ser Val Ala Arg Ile Pro Leu
                   85                  90                  95
   Pro Asn Leu Asn Glu Asp Leu Thr Cys Gly Asn Ile Leu Met Trp Glu
               100                 105                 110
   Ala Val Thr Leu Lys Thr Glu Val Leu Gly Val Thr Thr Leu Met Asn
           115                 120                 125
   Val His Ser Asn Gly Gln Ala Thr His Asp Asn Gly Ala Gly Lys Pro
       130                 135                 140
   Val Gln Gly Thr Ser Phe His Phe Phe Ser Val Gly Gly Glu Ala Leu
   145                 150                 155                 160
   Glu Leu Gln Gly Val Val Phe Asn Tyr Arg Thr Lys Tyr Pro Asp Gly
                   165                 170                 175
   Thr Ile Phe Pro Lys Asn Ala Thr Val Gln Ser Gln Val Met Asn Thr
               180                 185                 190
   Glu His Lys Ala Tyr Leu Asp Lys Asn Lys Ala Tyr Pro Val Glu Cys
           195                 200                 205
   Trp Val Pro Asp Pro Thr Arg Asn Glu Asn Thr Arg Tyr Phe Gly Thr
       210                 215                 220
   Leu Thr Gly Gly Glu Asn Val Pro Pro Val Leu His Ile Thr Asn Thr
   225                 230                 235                 240
   Ala Thr Thr Val Leu Leu Asp Glu Phe Gly Val Gly Pro Leu Cys Lys
                   245                 250                 255
   Gly Asp Asn Leu Tyr Leu Ser Ala Val Asp Val Cys Gly Met Phe Thr
               260                 265                 270
   Asn Arg Ser Gly Thr Gln Gln Trp Arg Gly Leu Ser Arg Tyr Phe Lys
           275                 280                 285
   Val Gln Leu Arg Lys Arg Arg Val Lys Asn Pro Tyr Pro Ile Ser Phe
       290                 295                 300
   Leu Leu Thr Asp Leu Ile Asn Arg Arg Thr Pro Arg Val Asp Gly Gln
   305                 310                 315                 320
   Pro Met Tyr Gly Met Asp Ala Gln Val Glu Glu Val Arg Val Phe Glu
                   325                 330                 335
   Gly Thr Glu Glu Leu Pro Gly Asp Pro Asp Met Met Arg Tyr Val Asp
               340                 345                 350
   Arg Tyr Gly Gln Leu Gln Thr Lys Met Leu
           355                 360
<210> 5
   <211> 21
   <212> PRT
   <213> Artificial
<220>
   <223> SV40
<220>
   <221> MISC_FEATURE
   <222> (1)..(21)
<400> 5
   Met Ala Pro Thr Lys Arg Lys Gly Ser Cys Pro Gly Ala Ala Pro Lys
   1               5                   10                  15
   Lys Pro Lys Glu Pro
               20
<210> 6
   <211> 21
   <212> PRT
   <213> Artificial
<220>
   <223> BKV
<220>
   <221> MISC_FEATURE
   <222> (1)..(21)
<400> 6
   Met Ala Pro Thr Lys Arg Lys Gly Glu Cys Pro Gly Ala Ala Pro Lys
   1               5                   10                  15
   Lys Pro Lys Glu Pro
               20
<210> 7
   <211> 9
   <212> PRT
   <213> Artificial
<220>
   <223> Any natural amino acid
<220>
   <221> MISC_FEATURE
   <222> (7)
<220>
   <223> Any natural amino acid
<220>
   <221> MISC_FEATURE
   <222> (8)
<400> 7
   Cys Pro Gly Ala Ala Pro Xaa Xaa Pro
   1               5

## Beschreibung

Die vorliegende Erfindung betrifft Zusammensetzungen aus virusähnlichen Partikeln für das Einbringen von RNA-Interferenz (RNAi-) induzierenden Molekülen in eukaryontische Zellen sowie Verfahren zur zelltypspezifischen Transduktion einer Vielzahl eukaryontischer Zellen mit RNAi-induzierenden Molekülen. Weiterhin betrifft die vorliegende Erfindung Verfahren zur Diagnose, Prävention und/oder Behandlung von Krankheiten oder Krankheitszuständen, die mit einer erhöhten Expressionsrate mindestens eines endogenen Gens und/oder mit der unerwünschten Expression mindestens eines endogenen Gens und/oder fremder Nukleinsäuren, insbesondere viraler Nukleinsäuren, einhergeht.

Bisher bekannte gentherapeutische Verfahren basieren auf der Transfektion von Zellen mit DNA-Konstrukten, welche für ein gewünschtes Genprodukt, zumeist ein therapeutisch wirksames Protein, kodieren. Die hierbei zum Einsatz kommenden Methoden sind in der Regel an in vitro Verfahren angepasst. Eine zielgerichtete, d.h. selektive Transfektion spezifischer Zelltypen ohne vorherige Isolation der Zellen aus ihrer natürlichen Umgebung ist mit den meisten aus dem Stand der Technik bekannten Verfahren, wie der Elektroporation oder Liposomentransfektion, nicht realisierbar.

Verfahren zum gewebsspezifischen DNA-Transfer *in vivo* beruhen bislang auf viralen Transfersystemen. Diese bergen jedoch aufgrund der potentiellen Rekombinationsgefahr mit zellulären Sequenzen ein kaum kalkulierbares Sicherheitsrisiko. Adenoviren und Adeno-assoziierte Viren, die derzeit favorisierten Systeme zum Transport von therapeutischen Genen, machen aufgrund ihrer hohen Immunogenität bei den meisten Patienten eine mehrmalige *in vivo*-Applikation unmöglich. Darüber hinaus ist es aufgrund des komplexen Aufbaus des Adenovirus und der Struktur des adenoviralen Genoms nur mit erheblichem Aufwand möglich, therapeutische DNA in ausreichendem Maße und in geeigneter Form am Zielort bereitzustellen.

Insbesondere im Hinblick auf eine unerwünschte Rekombination und die damit verbundene Gefahr, das Erbgut nachhaltig zu verändern, kamen gentherapeutische Verfahren zur Behandlung vorübergehend auftretender Krankheitsbilder wie beispielsweise akuter Infektionen nicht in Betracht. Nicht-virale Systeme, wie Liposomen und DNA-kondensierende Moleküle vermeiden zwar diese Nachteile, zeigen dafür allerdings, ähnlich wie retrovirale Systeme, weitaus geringere Transfereffizienzen und Zielzellspezifitäten.

Es besteht daher ein Bedarf, klinisch anwendbare gentherapeutische Zusammensetzungen und Verfahren zur Verfügung zu stellen, um diese Probleme zu umgehen.

Aufgabe der vorliegenden Erfindung war es, Zusammensetzungen bereitzustellen, die es bei gleichzeitiger Wahrung der Integrität des zellulären Genoms einer Zielzelle gestatten, gentherapeutisch wirksame Moleküle zelltypspezifisch und mit hoher Effizienz sowohl *in vivo* als auch *in vitro* in eukaryontische Zellen einzubringen. Eine weitere Aufgabe der vorliegenden Erfindung bestand darin, hocheffiziente Verfahren zur Behandlung von Infektionen mit Pathogenen oder zur Therapie und/oder Diagnose von Krankheitssymptomen, die mit einer veränderten Genexpression einhergehen, bereitzustellen.

Diese Aufgaben wurden erfindungsgemäß gelöst durch eine Zusammensetzung, umfassend ein virusähnliches Partikel ("virus like particle"; VLP), welches aus mehreren Molekülen mindestens eines viralen Kapsidproteins aufgebaut ist, dadurch gekennzeichnet, dass in dem VLP mindestens ein RNA-Interferenz (RNAi-)-induzierendes Molekül enthalten ist, wobei das Kapsidprotein ein modifiziertes JCV-VP1 Kapsidprotein ist, welches ein heterologes Kernlokalisierungssignal im Bereich der 25 N-terminalen Aminosäuren, enthält, wobei das heterologe Kernlokalisierungssignal die Aminosäuresequenz CPGAAPX1X2P (SEQ ID NO: 7) enthält, wobei X1; und X2 beliebige Aminosäuren und vorzugsweise jeweils K bedeuten. Die RNAi-induzierenden Moleküle bewirken nach dem Transfer in die Zielzelle eine wirksame Herabregulation der Expression eines Gens von Interesse. Weitere Gegenständer der Erfindung sind in den Ansprüchen beschrieben. RNA-Interferenz (RNAi) ist ein evolutionär konservierter Mechanismus zur Herabregulation der Expression eines oder mehrerer Gene ("gene silencing"). Eine Vielzahl eukaryontischer Organismen ist in der Lage, sich mittels RNAi gegen Viren und die Expression von Transposon-Elementen zu schützen. Das Prinzip der Herabregulation der Genexpression mittels RNAi basiert zum Einen auf dem sequenzspezifischen Abbau der durch Transkription eines Zielgens entstandenen RNA, insbesondere mRNA aufgrund einer Interaktion des Transkripts mit kurzen, z.B. 21-28 Nukleotide umfassenden RNA-Molekülen, so genannten kleinen interferierenden RNA-Molekülen ("small interfering RNA", siRNA). Der gezielte Abbau eines mRNA-Moleküls beginnt, katalysiert durch die RNase III Dicer, zunächst mit der Bildung von doppelsträngigen siRNA-Molekülen aus Vorläufermolekülen und einer anschließenden Prozessierung dieser Moleküle, gefolgt von der Hybridisierung eines Stranges der doppelsträngigen siRNA mit dem mRNA-Molekül unter Ausbildung eines doppelsträngigen siRNA-mRNA Hybridmoleküls. Anschließend findet eine Spaltung der mRNA innerhalb der mit der siRNA hybridisierten Region statt. Diese Spaltung bzw. Hydrolyse erfolgt zunächst endonukleolytisch, z.B. katalysiert durch die Endonuklease Argonaute 2 des RISC-Komplexes. Schließlich werden die dadurch entstandenen Spaltprodukte durch Exonukleasen des RISC-Komplexes hydrolysiert. Als Folge des gezielten Abbaus von mRNA-Transkripten wird die Expression des Zielgens zumindest teilweise unterdrückt.

Andererseits kann die Expression eines Gens auch auf translationeller Ebene herabreguliert werden. Die hierfür verantwortlichen Effektormoleküle werden als mikroRNA (miRNA) bezeichnet, welche ausgehend von einer Haarnadelstruktur ("hairpin") einer Vorläufer-RNA aus mehreren Prozessierungsschritten, an denen die Endonukleasen Drosha, Pasha und Dicer beteiligt sind, gebildet werden. miRNAs hemmen aufgrund ihrer teilweisen Komplementarität mit der Ziel-mRNA deren Translation.

Untersuchungen an Zellkulturen haben gezeigt, dass auch exogen bereitgestellte siRNA bzw. miRNA eine RNA-Interferenz in eukaryontischen Zellen, z.B. Säugerzellen, einschließlich humanen Zellen induzieren kann. Bisherige Verfahren zum Einbringen RNAi-induzierender Moleküle in eukaryontische Zellen erlauben es aufgrund der geringen Transfereffizienzen nicht oder nur sehr eingeschränkt, diesen Mechanismus als einen therapeutischen Ansatz zur Behandlung von Infektionen mit pathogenen Viren oder zur Behandlung von Krankheiten, die mit einer veränderten Genexpression einhergehen, zu nutzen.

Unter einem RNAi-induzierenden Molekül sind solche RNA-Moleküle zu verstehen, bei denen mindestens ein Polynukleotidstrang eine Sequenz aufweist, die ausreichend komplementär zu einer Ziel-RNA, bevorzugt zu einer Ziel-mRNA ist, um deren Prozessierung, d.h. deren Abbau zu bewirken. Um RNAi-induzierend zu wirken, ist es erforderlich, dass die Komplementarität zwischen RNAi-induzierendem Molekül und einer Region der Ziel-RNA ausreichend ist, um eine Hybridisierung und eine anschließende Prozessierung zu bewirken. Beispielsweise beträgt die Komplementarität mindestens 80 %, bevorzugt mindestens 90 % und am meisten bevorzugt mindestens 99 %, wobei an den 5'- und/oder 3'-Enden sowie an den Überhängen eines RNAi-Effektormoleküls auch nicht zur Ziel-RNA komplementäre Nukleotide vorliegen können.

Um RNAi zu induzieren, können verschiedene Wege eingeschlagen werden. Der direkte Transfer von Effektormolekülen, d.h. siRNA-Moleküle und/oder miRNA-Moleküle, stellt dabei eine Möglichkeit dar. Unter einem siRNA-Molekül ist vorzugsweise ein doppelsträngiges RNA Molekül mit einer Länge von 19-30 Nukleotiden, bevorzugt 20-28 Nukleotiden und besonders bevorzugt mit einer Länge von 21-23 Nukleotiden je Einzelstrang zu verstehen. siRNA-Moleküle bezeichnen ferner auch einzelsträngige RNA-Moleküle mit einer Länge von 19-30 Nukleotiden, bevorzugt 20-28 Nukleotiden und besonders bevorzugt mit einer Länge von 21-23 Nukleotiden, wobei das einzelsträngige RNA-Molekül zu mindestens 80 %, bevorzugt mindestens 90 % und insbesondere zu mehr als 99 % komplementär zu einer Sequenz einer Ziel-RNA, insbesondere einer Ziel-mRNA ist und eine Bindung der siRNA an die Ziel-RNA einen sequenzspezifischen Abbau bewirkt. Bevorzugt weisen siRNA-Moleküle 3'-seitig Überhänge von 1-3 Nukleotiden auf.

Als miRNA werden einzelsträngige RNAi-induzierende Moleküle mit einer Länge von 19-30 Nukleotiden, bevorzugt 20-28 Nukleotiden und besonders bevorzugt mit einer Länge von 21-23 Nukleotiden bezeichnet, die nach Hybridisierung mit einer Ziel-mRNA sowohl deren Translation inhibieren als auch deren Abbau bewirken können.

Alternativ ist es auch möglich, Vorläufermoleküle der eigentlich wirksamen Effektormoleküle, d.h. Vorläufermoleküle von siRNA und/oder miRNA bereitzustellen, die als Substrate für den siRNA/miRNA-Biogenese-Apparat der Zielzelle fungieren. Dazu zählen beispielsweise RNA-Vorläufermoleküle, wie doppelsträngige RNA (dsRNA) oder short hairpin RNA-Moleküle (shRNA), welche von den Endonukleasen wie Dicer, Drosha und/oder Pasha zu siRNA-Molekülen bzw. miRNA-Molekülen prozessiert werden. Hierzu können beispielsweise dsRNA-Moleküle oder short hairpin RNA-Moleküle (shRNA) mit einer Länge von mehr als 27 Nukleotiden, bevorzugt mehr als 30 Nukleotiden bis etwa 100 Nukleotiden oder länger und am meisten bevorzugt dsRNA-Moleküle mit einer Länge von 30-50 Nukleotiden verwendet werden.

Daneben ist es auch möglich, RNAi-induzierende Moleküle mittels eines DNA-basierten RNAi-Ansatzes in die Zielzellen einzubringen. Dafür werden DNA-Konstrukte kreiert, welche für dsRNA, shRNA, siRNA und/oder miRNA kodieren, wobei die kodierenden Elemente unter Kontrolle regulatorischer Elemente stehen, die eine Expression der dsRNA, shRNA, siRNA und/oder miRNA in der Zielzelle erlauben. Beispiele für solche Kontrollelemente sind Polymerase II-Promotoren oder Polymerase III-Promotoren, wie beispielsweise U6 oder H1.

Überraschend konnte nun festgestellt werden, dass mit der erfindungsgemäßen Zusammensetzung verschiedenste Arten von RNAi-induzierenden Molekülen zelltypspezifisch mit einer außerordentlich hohen Effizienz in eine Zielzelle eingebracht werden können. Die mit der erfindungsgemäßen Zusammensetzung eingebrachten RNAi-induzierenden Moleküle bewirken dabei eine Herabregulation der Expression eines Zielgens in einem Ausmaß, welches es nunmehr gestattet, den Mechanismus der RNA-Interferenz in der Therapie, Diagnostik und/oder Prävention von Krankheiten oder Krankheitszuständen, die durch die Expression einer Nukleinsäure eines pathogenen Organismus oder durch erhöhte oder unerwünschte Expression eines endogenen Gens verursacht werden, einzusetzen. Völlig unerwartet war dabei die Erkenntnis, dass mit der erfindungsgemäßen Zusammensetzung RNA-Moleküle mit definierter Sekundärstruktur in eine Zelle eingebracht werden können, wobei die eingebrachten RNA-Moleküle aufgrund ihrer Konformation von dem RNAi-Biogeneseapparat der Zielzelle als Substrat fungieren, so dass eine effiziente Herabregulation der Expression eines Zielgens möglich ist. Daher können mit der erfindungsgemäßen Zusammensetzung si/miRNA-VorläuferMoleküle wie dsRNA und shRNA derart in eine Zielzelle eingebracht werden, dass diese von den si/miRNA generierenden Enzymkomplexen in der Zelle als Substrat erkannt und effizient zu Effektormolekülen der sequenzspezifischen Herabregulation der Expression eines Zielgens prozessiert werden. Überraschend war zudem, dass auch VLP, welche aus DNA-Viren abgeleitet sind, für den Transfer von siRNA-bzw. miRNA oder deren Vorläufermolekülen wie dsRNA oder shRNA in eine Zielzelle eingesetzt werden können.

Unter einem RNAi-induzierenden Molekül sind ferner auch modifizierte RNA-Moleküle zu verstehen, welche 5'-seitig und/oder 3'-seitig Modifikationen, wie Fluoreszenzgruppen und/oder 3'-dTdT-Überhänge aufweisen und eine Herabregulation der Expression eines Gens von Interesse in einer eukaryontischen Zelle bewirken.

Unter einem RNAi-induzierenden Molekül sind gemäß der vorliegenden Erfindung zudem solche RNA-Moleküle zu verstehen, welche Analoga eines oder mehrerer Ribonukleotide in der Nukleotidsequenz aufweisen und eine Herabregulation der Expression eines Gens von Interesse in einer eukaryontischen Zelle bewirken. Derartige Ribonukleotid-Analoga können beispielsweise die Strukturstabilität des RNA-Moleküls oder die Stabilität gegenüber Ribonukleasen erhöhen. Ribonukleotid-Analoga sind dem Fachmann bekannt und sind gegenüber den ursprünglichen RNA-Molekülen durch Basenmodifikationen, Zuckermodifikationen, z.B. Modifikationen der 2'-OH Gruppe von Ribose und/oder Phosphatrückgratmodifikationen verändert.

Innerhalb der VLP kann ein einziger Typ eines RNAi-induzierenden Moleküls, d.h. siRNA, dsRNA, shRNA oder miRNA oder deren Vorläufermoleküle oder dafür kodierende DNA verpackt sein. Es können jedoch auch verschiedene Typen eines RNAi-induzierenden Moleküls in einem VLP eingebaut sein. Die RNAi-induzierenden Moleküle der erfindungsgemäßen Zusammensetzung können gegen ein oder mehrere Zielgene und/oder gegen die gleiche oder verschiedene Sequenzen eines einzigen Zielgens gerichtet sein.

Der Ausdruck "gegen ein Gen gerichtet sein" bedeutet in diesem Zusammenhang, dass ein RNAi-induzierendes Molekül die Sequenzinformation für einen sequenzspezifischen Abbau eines RNA-Moleküls, bevorzugt eines mRNA-Moleküls eines Zielgens enthält.

RNAi-induzierende Moleküle können erfindungsgemäß gegen ein oder mehrere Gene gerichtet sein, deren Expression herabreguliert werden soll. Bevorzugt sind die RNAi-induzierenden Moleküle der erfindungsgemäßen Zusammensetzung gegen aktiv exprimierte Gene gerichtet, deren Expression mit einem pathologischen Zustand korreliert.

Besonders bevorzugt enthält die erfindungsgemäße Zusammensetzung RNAi-induzierende Moleküle, die gegen mindestens ein Gen eines Pathogens, z.B. eines pathogenen Virus gerichtet sind. Weiterhin kann die erfindungsgemäße Zusammensetzung RNAi-induzierende Moleküle enthalten, die gegen mindestens ein endogenes Gen gerichtet sind, wobei die Expression des endogenen Gens und/oder die erhöhte Expression des endogenen Gens mit einem pathologischen Zustand korreliert. Beispiele für derartige zelluläre endogene Gene sind z.B. Tumor-assoziierte Gene, Autoimmun-assoziierte Gene, Stoffwechselerkrankungs-assoziierte Gene sowie insbesondere Gene, die mit neurodegenerativen und allgemeinen Nervenkrankheit assoziiert sind. Weitere Beispiele sind endogene Gene im Kontext infektiöser Erkrankungen (Wirtsfaktoren) sowie Gene im Kontext dystrophaler und progeroider Erkrankungen (allelspezifisch, z.B. Emerin, lamin A/C, FACE-1 etc.).

Das VLP der erfindungsgemäßen Zusammensetzung kann aus einem Typ eines Kapsidproteins oder aus mehreren verschiedenen Kapsidproteinen zusammengesetzt sein. Bevorzugt enthält die erfindungsgemäße Zusammensetzung ein VLP, das aus einem Typ eines Kapsidproteins zusammengesetzt ist. Besonders bevorzugt wird ein VLP verwendet, dessen Kapsidproteine die inhärente Eigenschaft aufweisen, unter geeignete Bedingungen sowohl *in vivo* als auch in vitro miteinander zu einem VLP zu assemblieren, d.h. für die Bildung des VLP aus den monomeren Proteinen werden keine weiteren Hilfsfaktoren benötigt.

Das Masseverhältnis von VLP zu RNAi-induzierendem Molekül liegt üblicherweise in einem Bereich zwischen 1:100 bis 100:1, bevorzugt 1:50 bis 50:1, besonders bevorzugt 1:20 bis 20:1 und am meisten bevorzugt in einem Bereich von 1:1 bis 20:1.

Bevorzugt wird ein VLP verwendet, das frei von anderen Komponenten des authentischen Virus, wie etwa authentischen viralen Nukleinsäuren ist. Besonders bevorzugt wird ein VLP verwendet, das aus dem Kapsidprotein VP1 des humanen JC-Virus (JCV) zusammengesetzt ist. JCV zählt zur Gattung der Polyoma Viren, deren virales Genom in Form doppelsträngiger DNA innerhalb des Kapsids vorliegt.

In einer besonderen Ausführungsform ist das VLP aus rekombinant hergestelltem Kapsidprotein aufgebaut. Der Begriff "Kapsidprotein" gemäß der vorliegenden Erfindung umfasst neben dem Kapsidprotein des Wildtypstammes des jeweiligen Virus, wie beispielsweise Wildtyp-VP1 auch modifizierte Formen des Kapsidproteins, d.h. Proteine, die sich vom Wildtyp-Kapsidprotein durch Mutationen wie etwa Substitutionen, Insertionen und/oder Deletionen unterscheiden. Zur Herstellung von rekombinantem VP1 verwendet man bevorzugt eine Nukleinsäure, welche die in SEQ ID NO. 1 gezeigte Sequenz, eine dieser Sequenz im Rahmen der Degeneration des genetischen Codes entsprechende Sequenz oder eine damit unter stringenten Bedingungen hybridisierende Sequenz umfasst, wobei man die Nukleinsäuresequenz oder einen diese Sequenz enthaltenden rekombinanten Vektor in eine geeignete Wirtszelle einbringt, die Wirtszelle unter Bedingungen kultiviert, bei denen eine Expression der Nukleinsäuresequenz erfolgt und das Protein aus der Zelle oder dem Zellüberstand isoliert. Stringente Hybridisierungsbedingungen sind vorzugsweise definiert gemäß Sambrook et al. (1989) Molecular Cloning. A Laboratory Manual, Second Edition, Cold Spring Harbor Laboratory Press, und umfassen einen Waschschritt von 30 min in 0,1 x SSC, 0,5 % SDS bei 68° C.

Die von der Nukleinsäure gemäß SEQ ID No.1 kodierte Aminosäuresequenz ist in SEQ ID No. 2 gezeigt. Im Rahmen der vorliegenden Erfindung wird vorzugsweise ein VP1- Polypeptid eingesetzt, welches SEQ ID No. 2 oder eine hierzu mindestens 70 %, vorzugsweise mindestens 80 %, besonders bevorzugt mindestens 90 % und am meisten bevorzugt mindestens 95 % identische Aminosäuresequenz enthält, wobei die Idendität über den gesamten Bereich von SEQ ID No. 2 bestimmt wird.

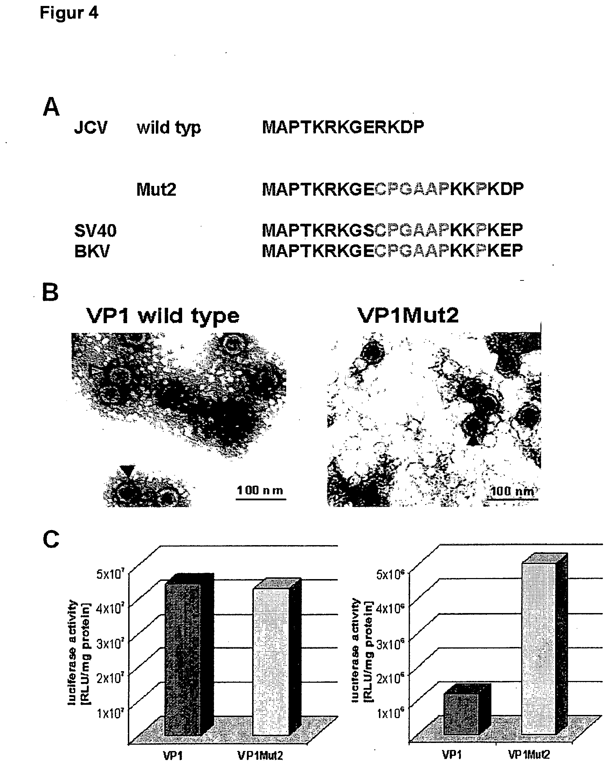

In einer weiteren bevorzugten Ausführungsform wird ein VP1 Protein verwendet, bei welchem die Aminosäuresequenz im N-terminalen Bereich, z.B. im Bereich der 25 N-terminalen Aminosäuren verändert wurde. Dabei wird vorzugsweise ein heterologes Kernlokalisierungssignal in die Aminosäuresequenz des VP1 eingeführt. Bevorzugte Kernlokalisierungssignale enthalten die Aminosäuresequenz CPGAAP X₁X₂P, wobei X₁ und X₂ beliebige Aminosäuren und vorzugsweise jeweils K bedeuten, und z.B. auf den Kernlokalisierungssignalen von SV40 oder BKV basieren. Die Aminosäuresequenzen besonders bevorzugter Kernlokalisierungssignale sind in Figur 4A gezeigt.

Die Nukleinsäuresequenz, die für ein bevorzugtes modifiziertes VP1 Protein (VP1-Mut2) kodiert, ist in SEQ ID No. 3 gezeigt. Die entsprechende Aminosäuresequenz ist in SEQ ID No. 4 gezeigt. Ein Gegenstand der vorliegenden Erfindung ist somit ein modifiziertes ICV-VP1 Protein, das ein heterologes Kernlokalisierungssignal, vorzugsweise wie oben angegeben, enthält, eine dafür kodierende Nukleinsäure und ein VLP, das zumindest ein entsprechendes modifiziertes VP1 Protein enthält, wobei das VLP gegebenenfalls Wirkstoffe, insbesondere Nukleinsäuren, aber auch andere Moleküle enthalten kann.

Die Isolierung rekombinanter Kapsidproteine erfolgt in Abhängigkeit des verwendeten Wirt/Vektorsystems direkt aus den Wirtszellen und/oder dem Zellkulturüberstand. Der Vorteil des rekombinanten Verfahrens liegt vor allem darin, dass VLPs auf einfache Weise in hoher Reinheit und in großen Mengen gewonnen werden können. Bei der rekombinanten Synthese von VP1 hat sich als Expressionssystem in der Praxis die Verwendung von Baculoviren in Verbindung mit Insektenzellen, z.B. mit der Insektenzelllinie Sf158, bewährt.

Die Eignung der VP1-VLPs als zellspezifisches DNA-Transport- und Transduktionssystem für Zellen renalen und neuronalen Ursprungs wurde bereits in DE 101 31 145.1 gezeigt. Dieses Dokument beschreibt ferner ein Verfahren zur Modifikation der VP1-VLPs, um deren Zelltropismus gezielt zu verändern, so dass selektiv ganz bestimmte Zielzellen und Gewebe transduziert werden können.

Überraschend wurde nun herausgefunden, dass innerhalb eines VLP des JC-Virus auch RNAi-induzierende Moleküle, insbesondere dsRNA, shRNA, miRNAs, siRNA oder deren Vorläufermoleküle verpackt werden können, ohne dabei die Struktur dieser Moleküle zu verändern. Es wurde festgestellt, dass RNAi-induzierende Moleküle, welche in den Zielzellen RNAi induzieren, innerhalb der VLP sowohl vor einem Abbau als auch vor strukturellen Veränderungen hinreichend geschützt sind. Unter dem Begriff Zielzellen sind eukaryontische Zellen und hierbei bevorzugt Zellen eines mehrzelligen Organismus, insbesondere Säugerzellen, einschließlich Zellen menschlichen Ursprungs zu verstehen.

Für das Einbringen von RNAi-induzierenden Molekülen in Zellen, welche dem natürlichen Wirtsspektrum des Virus entsprechen, dessen Kapsidprotein Bestandteil der erfindungsgemäßen Zusammensetzung ist, wird bevorzugt ein VLP verwendet, dessen Kapsidprotein dem Wildtyp-Kapsidprotein entspricht. Beispielsweise umfasst das Wirtsspektrum des JCV Zellen des Neuralgewebes und Zellen neural verwandter Gewebe, wie beispielsweise Oligodendrozyten, Astrozyten und Gliazellen. Sollen in diese Zellen RNAi-induzierende Moleküle eingebracht werden, enthält die erfindungsgemäße Zusammensetzung bevorzugt ein VLP, welches aus dem Wildtyp-VP1 Kapsidprotein des JC-Virus aufgebaut ist.

Für die Bereitstellung von RNAi-induzierenden Molekülen für Zellen, die nicht dem natürlichen Wirtsspektrum des Virus entsprechen, dessen Kapsidprotein Bestandteil der erfindungsgemäßen Zusammensetzung ist, wird bevorzugt ein VLP verwendet, dessen Kapsidprotein gegenüber dem Wildtyp-Kapsidprotein modifiziert ist. Die Verwendung modifizierter VLP innerhalb der erfindungsgemäßen Zusammensetzung ermöglicht es vorteilhaft, Erkrankungen zu behandeln, die auf bestimmte Zelltypen bzw. auf bestimmte Gewebe beschränkt sind. Besonders bevorzugt besteht die erfindungsgemäße Zusammensetzung aus einem VLP des JC-Virus, wobei mindestens ein Kapsidprotein des VP1-VLP gegenüber dem Wildtyp-VP1 modifiziert ist.

Modifikationen umfassen zum Einen Änderungen in der Aminosäuresequenz des Wildtyp-Kapsidproteins. Dazu zählen insbesondere Insertionen, Deletionen, Substitutionen und/oder Fusionen mit anderen Aminosäuresequenzen unter der Voraussetzung, dass die inhärente Eigenschaft der Kapsidproteine zur Selbstassemblierung, d.h. zur Ausbildung von VLP, nicht verloren geht. So kann das VLP der erfindungsgemäßen Zusammensetzung ein oder mehrere zusätzliche heterologe Proteine oder Proteinanteile in der Kapsidstruktur in Form eines Fusionsproteins aufweisen. Darunter ist zu verstehen, dass ein heterologes Protein oder ein heterologer Proteinanteil in der Kapsidstruktur verankert ist, wobei bevorzugt mindestens ein Teil dieses Proteins von außen zugänglich ist. VLPs, die aus solchen Fusionsproteinen zusammengesetzt sind, können aufgrund des Fusionsanteils mit einem spezifischen Oberflächenrezeptor der beabsichtigten Zielzelle interagieren. Auf diese Weise kann die Spezifität der Interaktion mit den vorgesehenen Zielzellen gewährleistet und in Abhängigkeit von der Anwendung auf eine Vielzahl an Zelltypen angepasst werden. Durch eine gezielte Veränderung der Kapsidproteine ist es also möglich, den Tropismus der VLP auf verschiedene Zellen oder Gewebe zu beschränken.

Eine weitere Möglichkeit, den Tropismus der VLP auf einen definierten Zelltyp zu richten, besteht darin, die VLP der erfindungsgemäßen Zusammensetzung chemisch zu modifizieren. Durch chemische Modifikation der VLP ist es möglich, die erfindungsgemäße Zusammensetzung flexibel als Transfersystem für RNAi-induzierende Moleküle für bestimmte Zellen bzw. Gewebe einzusetzen. Für die gezielte Veränderung der Zielzellspezifität der VLP haben sich Konjugate aus VLP und einem zelltypspezifischen Ligand als vorteilhaft erwiesen. In einer bevorzugten Ausführungsform ist mindestens ein Ligand mit dem VLP assoziiert. Der Ligand kann dabei grundsätzlich jede beliebige Substanz sein.

Beispielsweise kann der Ligand eine zielzellspezifische Gruppe sein, z.B. ein Bindungspartner für einen Zelloberflächenrezeptor. Geeignete Beispiele für Bindungspartner sind natürliche Liganden oder synthetische Analoga davon, wobei hochmolekulare Liganden wie Proteine, z.B. Transferrin, Antikörper oder Zucker wie Mannose, aber auch niedermolekulare synthetische Liganden, z.B. das Tripeptid-Motiv R-G-D (Arg-Gly-Asp), eingesetzt werden können. Alternativ oder zusätzlich kann als Ligand auch eine Markierungsgruppe, z.B. eine durch geeignete Nachweismethoden erkennbare Gruppe, wie etwa eine Fluoreszenzmarkierungsgruppe oder Biotin, verwendet werden. Weiterhin kann der Ligand auch eine Effektorgruppe, z.B. eine cytotoxische Gruppe, sein. Selbstverständlich können auch Kombinationen mehrerer Liganden, insbesondere Kombinationen der zuvor genannten Liganden, eingesetzt werden.

Die Wechselwirkungen zwischen Ligand und dem Kapsidprotein des VLP werden bevorzugt über chemische Ankergruppen vermittelt. Dabei hat sich insbesondere die Beladung der Kapsidproteine mit kationischen Polymeren als Ankermolekül für zellspezifische Liganden als geeignet erwiesen.

Spezifische Beispiele für kationische Polymere sind auf im Wesentlichen basischen Aminosäuren basierende Polymere, wie etwa Polylysin, insbesondere Poly-L-Lysin etc. Weitere spezifische Beispiele für geeignete kationische Polymere sind Polyalkylenamine, Polyalkylenimine, vorzugsweise Poly-C₂-C₄-Alkylenimine, insbesondere Polyethylenimin (PEI), pAMAM (Polyamidoamin)-Dendrimere und fraktionierte Dendrimere sowie kationisch modifiziertes Polyethylenglykol. Polyethylenimin ist ein besonders bevorzugtes kationisches Polymer im Sinne der vorliegenden Erfindung, da es nicht toxisch ist und eine hohe Dichte an positiven Ladungen aufweist. PEI ist weiterhin dazu in der Lage, nach Aufnahme in die Zellen eine pHabhängige Strukturveränderung zu bewirken, die zur Destabilisierung von endosomalen und lysosomalen Zellkompartimenten führt und somit die Freisetzung von RNAi-induzierenden Molekülen in das Zytoplasma erleichtert. Dieser Prozess wird durch die ausgeprägte Pufferkapazität der Iminogruppen unterstützt, die nach Azidifizierung in den Lysosomen protoniert werden und dann zu einer osmotischen Ruptur der Vesikelmembran führen.

Das Gewichtsverhältnis von VLP zu kationischem Polymer in den Konjugaten kann in breiten Bereichen Variiert werden. So haben sich Gewichtsverhältnisse von 5:1 bis 1:10 als geeignet erwiesen, wobei Gewichtsverhältnisse von 2:1 und 1:5 besonders bevorzugt sind, um eine optimale Bindung zu ermöglichen.

Verfahren zur Herstellung von Konjugaten aus VP1-VLP und kationischen Polymeren sind aus DE 101 31 145.1 bekannt.

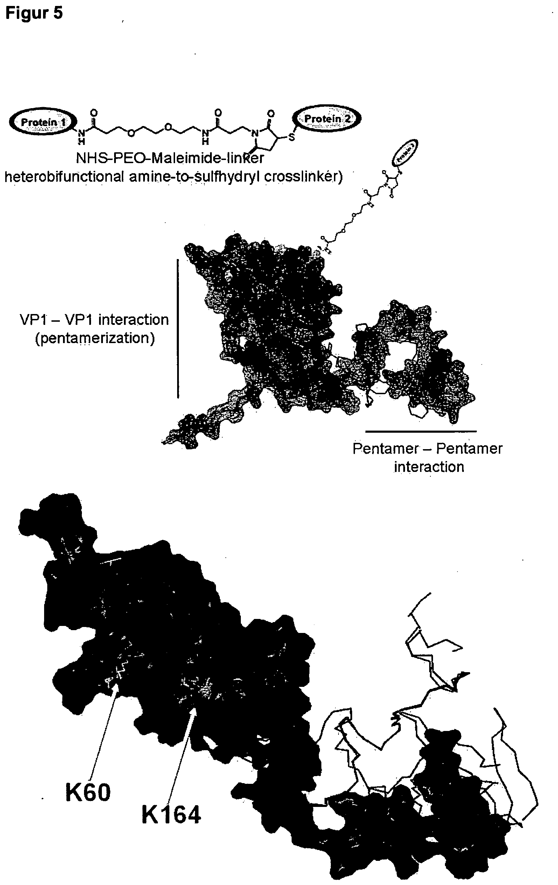

In einer weiteren bevorzugten Ausführungsform können heterologe Bindepartner bzw. Liganden, z.B. Polypeptide oder Markierungsgruppen, direkt und mit kontrollierbarer Stöchiometrie an die VLPs gekoppelt werden. Das Beispiel eines hetero-bifunktionalen Protein-Protein Linkers ist in Figur 5 gezeigt. Hierbei wird die Aminogruppe von Lysinresten an der Außenseite, vorzugsweise von K60 und/oder K164 benutzt, um den Linker (hier einen PEO-Maleimid Linker) an VP1 zu binden. Liganden können durch einen Cystinrest, z.B. einen carboxyterminalen Cystinrest an die zweite aktive Stelle des Linkers binden und somit eine stabile VP1-Ligand Konjugation ergeben, die eine VLP-Ladung gezielt in die ligandenspezifischen Zelltypen liefern kann.

Noch ein Gegenstand der Erfindung ist somit ein JCV-VP1 Kapsidprotein, an das mindestens ein heterologer Bindepartner, z.B. ein Polypeptid, über die Aminosäurereste K60 und/oder K164 gebunden ist.

Gegenstand der vorliegenden Erfindung sind weiterhin Zusammensetzungen umfassend
(i) ein Konjugat aus einem virusähnlichen Partikel (VLP), das aus mehreren Molekülen mindestens eines viralen Kapsidproteins aufgebaut ist und mindestens einem zelltypspezifischen Ligand, wobei VLP und Ligand bevorzugt über Ankergruppierungen, wie beispielsweise kationische Polymere oder über heterobifunktionale Linker, miteinander verbunden sind und
(ii) RNAi-induzierende Moleküle, welche in dem Konjugat aus VLP und Ligand eingebaut sind.

Bevorzugt wird ein VLP verwendet, das aus dem Kapsidprotein VP1 des humanen Polyomavirus JC abgeleitet ist. Bevorzugt werden als RNAi-induzierende Moleküle solche verwendet, wie oben beschrieben.

Zur Herstellung einer Zusammensetzung gemäß dem Oberbegriff des Anspruchs 1 werden die RNAi-induzierenden Moleküle und gegebenenfalls weitere Wirksubstanzen sowie Träger-, Hilfs- und/oder Zusatzstoffe in der gewünschten Menge bzw. Konzentration vor und/oder während der Assemblierung den Kapsidproteinen zugesetzt. Auf diese Weise können VLP entstehen, welche im Inneren der Partikel RNAi-induzierende Moleküle enthalten. Bevorzugt erfolgt die Beladung der VLP mit RNAi-induzierenden Molekülen während der Assoziation der Kapsidproteine zu dem VLP. Das Verpacken der RNAi-induzierenden Moleküle kann sowohl *in vitro* als auch *in vivo* erfolgen, wobei die Inkorporation der RNAi-induzierenden Moleküle in das VLP bevorzugt unter in vitro Bedingungen erfolgt. Die Inkorporation RNAi-induzierender Moleküle in das Innere der Kapsidhülle kann z.B. durch Dissoziation (Deassemblierung) der Kapsidhülle und anschließende Reassoziation (Reassemblierung) in Gegenwart der RNAi-induzierenden Moleküle oder durch osmotischen Schock der VLP in Gegenwart der RNAi-induzierenden Moleküle erfolgen. An das mit RNAi-induzierenden Molekülen beladene VLP kann weiterhin eine zielzellspezifische Gruppe, die mit Rezeptoren auf der Oberfläche einer Zielzelle bindefähig ist, wie oben beschrieben, angebunden werden. Die Bedingungen für die Beladung des VLP sind jeweils so zu wählen, dass ein Abbau bzw. eine Strukturveränderung der RNAi-induzierenden Moleküle vermieden wird.

Bei der Verpackung kann das Verhältnis von Kapsid-Protein-Monomer zu RNAi-induzierendem Moleküle in Abhängigkeit von der Anzahl der KapsidProteine je VLP und der Größe der zu verpackenden Moleküle über einen weiten Bereich variieren. Das Masseverhältnis von Kapsid-Protein zu RNAi-induzierendem Molekül liegt üblicherweise in einem Bereich zwischen 1:100 bis 100:1, bevorzugt 1:50 bis 50:1, besonders bevorzugt 1:20 bis 20:1 und am meisten bevorzugt in etwa 1:1 bis 20:1.

Vor dem Hintergrund, dass das Kapsidprotein rekombinant und getrennt von den RNAi-induzierenden Molekülen in großen Mengen und hoher Reinheit produziert wird und die Verpackung der Nukleinsäuren nicht wie bei retroviralen, Adeno-assoziierten oder Adeno-viralen Vektoren in Verpackungszelllinien erfolgt, lassen sich Kontaminationen mit viralen Nukleinsäuren und die potentielle Gefahr der Entstehung infektiöser Viren aufgrund von Rekombinationsereignissen ausschließen. Da weiterhin die Verpackung RNAi-induzierender Moleküle und die Beladung der VLP mit zellspezifischen Liganden unter definierten in vitro Bedingungen erfolgt, stellt das VLP-RNAi-Transfersystem und hierbei insbesondere das VP1-VLP-Transfersystem eine biologisch sichere Plattformtechnologie dar, die die Vorteile viraler und nicht-viraler Systeme vereinigt, ohne allerdings deren Nachteile aufzuweisen. So zeichnen sich die VLP der erfindungsgemäßen Zusammensetzung insbesondere dadurch aus, dass sie frei von Nukleinsäuren des ursprünglichen Virus sind. Derartige VLP, insbesondere VLPs aus rekombinanten VP1-Molekülen, sind in WO 97/19174 beschrieben.

Die erfindungsgemäße Zusammensetzung kann verwendet werden, um RNAi-induzierende Moleküle spezifisch in beliebige Zelltypen sowohl *in vivo* als auch *in vitro* einzubringen. Der zielgerichtete Transfer funktioneller RNAi-induzierender Moleküle führt dabei innerhalb eines mehrzelligen Organismus zu einer lokal begrenzten Herabregulation der Expression eines Gens von Interesse mittels RNAi. So kann bei Verwendung der erfindungsgemäßen Zusammensetzung beispielsweise die erhöhte Expression eines endogenen Gens transient herabreguliert werden, ohne dass die Gefahr einer Veränderung des Genoms der Zielzelle besteht. Es ist hierbei jedoch auch möglich, die genetische Information für den zielgerichteten, d.h. sequenzspezifischen Abbau von RNA, insbesondere mRNA mittels RNAi dauerhaft in eine Zielzelle zu integrieren. Weiterhin können die erfindungsgemäßen Zusammensetzungen dazu verwendet werden, um zelltypspezifisch die Expression unerwünschter Gene, beispielsweise als Folge einer Transposition von DNA-Abschnitten oder einer viralen Infektion herunterzuregulieren. Insbesondere kann die erfindungsgemäße Zusammensetzung dazu verwendet werden, um die Expression mindestens eines Gens, welche mit einem pathologischen Zustand korreliert, herabzuregulieren.

Eine erfindungsgemäße Zusammensetzung kann dazu verwendet werden, um chronische Krankheiten bzw. deren Symptome zu behandeln. In diesem Fall können RNAi-induzierende Moleküle beispielsweise in Form von DNA-Konstrukten verwendet, die eine dauerhafte Bereitstellung von siRNA oder deren Vorläufermolekülen in einer Zielzelle bewirken. Weiterhin ist es mit der erfindungsgemäßen Zusammensetzung nunmehr möglich, transiente Gentherapien beispielsweise zur Behandlung akuter Infektionen sowohl in der Veterinär- als auch in der Humanmedizin zelltypspezifisch zu etablieren. Die zu verabreichenden Mengen an der erfindungsgemäßen Zusammensetzung sind u.a. von der Art der Erkrankung, der Schwere der Symptome und von dem Umfang der betroffenen Zellen abhängig.

Die erfindungsgemäße Zusammensetzung kann lokal oder systemisch nach bekannten Verfahren zur Applikation von VLPs verabreicht werden.

Ein weiterer Gegenstand der vorliegenden Erfindung ist daher ein Verfahren zum Einbringen RNAi-induzierender Moleküle in eine Zielzelle, umfassend die Schritte
(i) Assemblieren der viralen Kapsidproteine zu VLP in Anwesenheit der RNAi-induzierenden Moleküle und
(ii) Inkontaktbringen des mit den RNAi-induzierenden Molekülen beladenen VLP mit der Zielzelle unter Bedingungen, bei denen eine Aufnahme der RNAi-induzierenden Moleküle in die Zielzelle erfolgen kann.

Zur Veränderung der Zielzellspezifität kann weiterhin an das aus Schritt (i) erhaltene VLP eine oder mehrere zielzellspezifische Gruppen angebunden werden, welche mit Rezeptoren auf der Oberfläche einer Zielzelle bindefähig sind. Das erfindungsgemäße Verfahren kann unter anderem zur Herabregulation mindestens eines Gens von Interesse in einer Zielzelle eingesetzt werden.

Als Zielzelle kommt jede eukaryontische Zelle in Betracht. Bevorzugt ist die eukaryontische Zelle eine Säugerzelle und insbesondere menschlichen Ursprungs. Mit einer Zusammensetzung gemäß dem Oberbegriff des Anspruchs 1 ist es möglich, RNAi-induzierende Moleküle sowohl in Zellen einzubringen, die sich innerhalb ihrer natürlichen Umgebung befinden als auch in Zellen, die aus ihrem natürlichen Kontext isoliert worden sind.

Entsprechen die Zielzellen, in welche RNAi-induzierende Moleküle eingebracht werden sollen, nicht dem natürlichen Wirtsspektrum der Viren, aus denen die im erfindungsgemäßen Verfahren verwendeten VLPs abgeleitet sind, kann die Zelltypspezifität der VLPs vor, während oder nach der Beladung mit RNAi-induzierenden Molekülen und gegebenenfalls weiteren Wirksubstanzen durch Komplexierung der Kapsidproteine mit Liganden wie oben beschrieben, verändert werden ("Retargeting"). Dabei werden vorteilhaft solche Liganden gewählt, die speziell mit Rezeptoren bindefähig sind, welche auf der Zelloberfläche der Zielzellen exponiert werden. Alternativ können hierfür VLP verwendet werden, die aus Kapsidproteinen bestehen, welche in ihrer Aminosäurezusammensetzung ein heterologes Protein aufweisen, dass einen zielgerichteten Transfer von RNAi-induzierenden Molekülen und gegebenenfalls weiteren Wirksubstanzen aufgrund der Bindefähigkeit an spezifische Rezeptoren auf der Zellaußenseite der Zielzellen vermittelt.

Ein weiterer Gegenstand der vorliegenden Erfindung ist eine pharmazeutische Zusammensetzung, umfassend VLP wie oben beschrieben, in welcher RNAi-induzierende Moleküle wie oben beschrieben inkorporiert sind, zur Diagnose, Prävention und/oder Behandlung von Krankheiten oder Krankheitszuständen, welche durch Expression einer fremden Nukleinsäure, beispielsweise einer Nukleinsäure eines pathogenen Organismus, insbesondere eines pathogenen Virus, oder durch erhöhte oder unerwünschte Expression eines endogenen Gens verursacht werden.

Weiterhin betrifft die vorliegende Erfindung einen Testkit zum Einbringen von RNAi-induzierenden Molekülen in beliebige Zellen bzw. Gewebe, umfassend VLP wie oben beschrieben.

Noch ein weiterer Gegenstand der Erfindung sind VLP-Zusammensetzungen wie oben genannt, die andere Arten von Nukleinsäuren enthalten, z.B. immunstimulatorische Nukleinsäuren, ggf. in Kombination mit Polypeptid- oder Peptid-Immunogenen, Aptamere oder siDNA-Moleküle. Weitere geeignete Wirkstoffe sind polare Cytostatika oder Toxine.

Neben den zuvor genannten RNAi induzierenden Molekülen können insbesondere im Kontext infektiöser retroviraler Erkrankungen (z.B. HIV-1) auch andere Nukleinsäuren weitere therapeutische wie prophylaktische VLP Ladungen zu erwähnen. Zur prophylaktischen Anwendungen seien hier immunstimulatorische Sequenzen erwähnt (ISS), möglichst in Kombination mit Immunogenen des betreffenden Retrovirus (z.B. Env-Komponenten aus HIV-1). Zudem soll zur Therapie eine Virusinhibition durch verschiedene, nicht RNAi induzierende Nukleinsäuresequenzen erreicht werden. Diese Moleküle können aus inhibitorischen DNA und RNA Aptamere ausgewählt werden, ebenso wie aus sogenannten siDNAs, die durch eine vorzeitige Aktivierung des Enzyms RNAseH das Genom von Retroviren vor der Integration in das Wirtsgenom zerstören können. (Matskevich et al., Aids Res & Human Retroviruses 22 (2006), 1220-1230; Matzen et al. Nature Biotechnology 6 (2007)). Hinsichtlich des Einschlusses solcher Nukleinsäuren in VLPs wird auf die Ausführungen im Zusammenhang mit RNAi Molekülen Bezug genommen.

Die Erfindung wird nunmehr durch die folgenden Beispiele, sowie die beigefügten Figuren und das Sequenzprotokoll weiter erläutert.

### Beispiel 1:

### Material und Methoden

### Konstruktion und Synthese von siRNA-Molekülen

RNA-Oligonukleotide wurden chemisch synthetisiert und von Dharmacon (Lafayette, Colorado) bezogen. Alle siRNA-Moleküle enthielten 3'-dTdT-Überhänge. Fluoreszenzmarker wurden an das 5'-Ende des Sinn-(sense-) Stranges der siRNA-Moleküle gekoppelt, um deren Funktionalität nicht zu beeinflussen. Nukleotide (dNTPs) für eine PCR wurden von Boehringer (Mannheim, Deutschland) bezogen, PCR-Primer und DNA-Fragmente zur Herstellung von shRNA mittels *in vitro-* Transkription wurden von NAPS (Göttingen, Deutschland) bereitgestellt. Doppelsträngige RNA mit einer Länge von 27 Nukleotiden (27mer dsRNA) wurde chemisch synthetisiert und von IBA (Göttingen, Deutschland) bereitgestellt.

**Tabelle 1: siRNA-Moleküle, Zielgene, Positionen, Modifikationen**

| **siRNA** | **Zielgen** | **Nukleotid-position** | **Modifikationen** | **Anmerkungen** |
|---|---|---|---|---|
| **siLamA1** | humanes Lamin A/C | 608-630 | 3'-dTdT Überhänge | |
| **siLamA2** | humanes Lamin A/C | 672-694 | 3'-dTdT Überhänge | |
| **siL27AC** | humanes Lamin A/C | 670-697 | 5'-phosphoryliert, keine dTdT-Überhänge | Stumpfendige 27mer dsRNA |
| **siLamA3** | humanes Lamin A | 1919-1941 | 3'-dTdT Überhänge | |
| **siLamA-F** | humanes Lamin A/C | | 5'-sense FITC 3'-dTdT-Überhänge | Fluoreszenzfarbstoff (Fluorescein) ist kovalent an das 5'-Ende des Passagierstranges gekoppelt |
| **siEg5-F** | humanes Eg5 | 1547-1569 | 5'-sense Rhodamin 3'-dTdT-Überhänge | Fluoreszenzfarbstoff (Rhodamin rot) ist kovalent an das 5'-Ende des Passagierstranges gekoppelt |
| **siEme1** | humanes Emerin | 628-640 | 3'-dTdT-Überhänge | |
| **siEme2** | humanes Emerin | | 3'-dTdT-Überhänge | |
| **siEme3** | humanes Emerin | | 3'-dTdT-Überhänge | |
| **siGL2** | Leuchtkäfer-Luziferase | 153-175 | 3'-dTdT-Überhänge | nichtverwandte Kontrolle |

### Antikörper und indirekte Immunfluoreszenzmikroskopie

Transduzierte Zellen wurden 44 Stunden nach der Behandlung mit -20° C kaltem Methanol mit VLP behandelt. Als Primärantikörper wurden ein Maus-anti-Lamin A/C-Antikörper (Klon 636.23), ein Maus-anti-Emerin-Antikörper (Novagen) und ein Maus-anti-α-Tubulin-Antikörper (D1H, Sigma, Deutschland) verwendet. Als Sekundärantikörper wurden Rhodamin- oder Fluorescein-konjugierte Ziege anti-Maus-IgGs eingesetzt. Nach dem Fixieren wurden die mit PBS gewaschenen Zellen für eine Stunde bei 37° C in einer Feuchtekammer mit einem ersten Antikörper inkubiert. Anschließend wurden die Zellen dreimal mit PBS gewaschen und fluoreszierende Sekundärantikörper wurden für eine Stunde bei 37° C zugesetzt. Ungebundene Antikörper wurden durch dreimaliges Waschen mit PBS entfernt und anschließend wurde die DNA mit 2 µM Hoechst 33342 Farbstoff (Hoechst, Deutschland) visualisiert. Hierzu wurden die Zellen mit Mowiol (Hoechst, Deutschland) auf Objektträger fixiert.

### Quantitative Western Blot-Analyse

Eine SDS-Gelelektrophorese wurde gemäß den Standardprotokollen durchgeführt. Proteine wurden mittels Standard-SDS-Gelelektrophorese aufgetrennt und anschließend unter Verwendung des Semi-dry-Transferverfahrens auf eine Nitrozellulose-Membran transferiert. Membranen wurden in TBST (20 mM Tris-HCI, 150 mM NaCl, 0,2 % Tween 20, pH 7,4) blockiert, wobei das TBST 5 % Magermilchpulver enthielt. Antikörper gegen Lamin A/C, Emerin oder Vimentin wurden ebenfalls in TBST verdünnt, wobei TBST hierbei 2,5 % Magermilchpulver enthielt. Die so verdünnten Antikörper wurden mit der Membran für 1 h bei Raumtemperatur inkubiert. Die Vimentin-Proteingehalt wurden bestimmt, um jeweils gleiche Proteinmengen im Western Blot analysieren zu können. Die Membranen wurden zweimal mit TBST und einmal mit TBST plus 0,5 % Triton X-100 gewaschen. Affinitätsgereinigte Meerrettich konjugierte Schwein anti-Maus-Immunglobuline wurden von Dako (Kopenhagen, Dänemark) bezogen. Sie wurden 1:10.000 in Blockierungspuffer, welcher 2,5 % Magermilchpulver enthielt, verdünnt und für 2 h bei Raumtemperatur mit der Membran inkubiert. Die Banden wurden unter Verwendung des ECL-Kits (Amersham Biosciences) detektiert und auf einem Lumilmager (Boehringer/Roche, Deutschland) quantifiziert.

### Zellen

**Tabelle 2: In dieser Studie verwendete Zelllinien**

| **Zelllinien** | Herkunft (Quelle) |
|---|---|
| **HeLa SS6:** | Humanes Cervixadenokarzinom (ATCC und Gey et al., 1952) |
| **MCF-7:** | Humanes Brustadenokarzinom (ATCC und Soule et al., 1973) |
| **293T** | Human Embryoniere (ATCC) |
| **CÖS-7:** | Nierenfibroblasten der Afrikanischen Gelbgrünen Meerkatze (ATCC) |

| **Humane Chondrocyten** | |
|---|---|
| **Glioma SW103** | Humane Glioblastomzelllinie (ATCC |
| **HeLa S3** | Humanes Cervixadenokarzinom, auf das Wachstum in Suspension adaptiert |
| **SupT1** | Humane T-Zelllinie (ATCC) |

Adhärente Zellen (d.h. HeLa SS6, MCF-7, 293T, COS-7, Chondrocyten und Glioma SW103) wurden in DMEM, welches mit 10 % fötalem Kälberserum (FCS) und Antibiotika (Penicillin und Streptomycin, pen/strep) supplementiert war, kultiviert. Zellen in Suspension (d.h. HeLa S3 und SupT1) wurden in RPMI-1640 High glucose-Medium, welches mit 10 % FCS und pen/strep supplementiert war, kultiviert.

### Herstellung der VLP und Beladung

VP1-Protein des Polyoma-JC-Virus wurde mittels eines baculoviralen Systems in Insektenzellen (SF) exprimiert. Sezerniertes Protein wurde aus dem Mediumüberstand mittels Grädientenzentrifugation oder lonenaustausch-FPLC isoliert.

Aufgereinigtes VP1 lag in Form von Pentameren und höher geordneten Kapsiden vor, so dass diese vor dem Beladen mit einer Nukleinsäure zunächst mit einem Deassemblierungspuffer (10 mM Tris, pH 7,5, 10 mM EGTA, 150 mM NaCl, 5 mM DTT) zunächst deassembliert werden mussten. Das Beladen der VLPs durch Reassemblierung erfolgte mittels Dialyse der VP1/Nukleinsäuremischung gegen Reassemblierungspuffer (10 mM Tris, pH 7,5, 1 mM CaCl₂, 150 mM NaCl) in einem Mikrodialyseansatz mit einer Ausschlussgröße von 3,5 kDa über Nacht bei 4° C. Das Standardverhältnis von DNA zu VLP betrug 1:10 (z.B. wurden 500 ng zu 5 µg VLP zugesetzt). Für das anschließende Retargeting wurden die reassoziierten VLPs mit PEI-Tf in einem Verhältnis von 1:5 (PEI zu VLP, z.B. wird 1 µg PEI mit 5 µg VLP versetzt) miteinander vermischt.

### Transfektion/Transduktion

Zellen wurden in 24-Well-Platten zu je 50.000 bis 100.000 Zellen/ml 24 Stunden vor der Behandlung mit siRNA-beladenen VLP ausplattiert. Im Falle der in Suspension kultivierten Zellen (d.h. SupT1- und HeLa S3-Zelllinien) wurde das Kulturmedium mit 0,5 ng/ml Desfferioxaminase ersetzt, um den Transferrinmetabolismus zu stimulieren und die Transferrinrezeptordichte auf der Zelloberfläche zu erhöhen. Unmittelbar vor der Behandlung mit VLP wurde das Stimulationsmedium durch normales Kulturmedium ersetzt, um cytotoxische Nebeneffekte des Mittels zu vermeiden.

### Ergebnisse

### Einbringen von DNA and siRNA in humane Gliomazellen

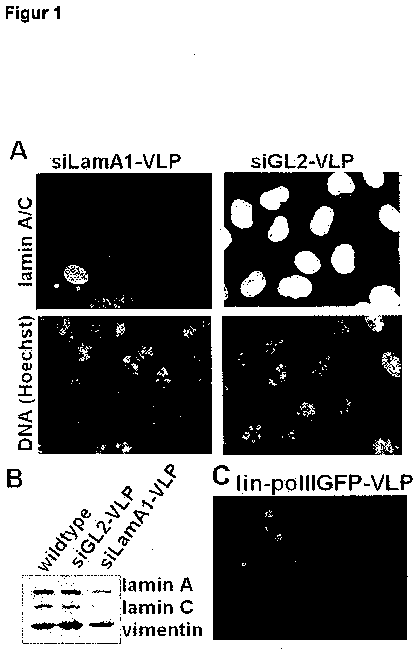

Dem Kulturmedium humaner Glioma SW103-Zellen wurden siRNA beladene VLP zugesetzt. Eine Stunde nach der Behandlung konnten fluoreszenzmarkierte siRNAs, welche gegen humanes Lamin A/C (siLamA-F) gerichtet waren, im Zytoplasma der Gliomazellen beobachtet werden (siehe Fig. 1). Die spezifische Aufnahme von ausschließlich VLP-verpackter siRNA wurde durch die Behandlung von Kontrollzellen mit nackter siRNA und leeren VLP bestätigt. 24 Stunden nach der VLP-Transduktion wurden die Zellen im Hinblick auf RNA-Interferenzeffekte mittels indirekter Immunfluoreszenzmikroskopie und Western Blot-Analyse untersucht. Die Mengen des Zielproteins Lamin A/C wurden in den mit siLamA-F-VLP behandelten Zellen wirksam herabreguliert, wobei dies in den Kontrollzellen nicht beobachtet werden konnte (Fig. 1).

Das Einbringen von siRNA-Molekülen in Glioma SW103-Zellen wurde ebenfalls als eine Qualitätskontrolle für das Verpacken von Nukleinsäuren in VLP in nachfolgenden Experimenten, einschließlich des Retargetings der VLP durch Komplexierung dieser mit PEI-Tf, verwendet.

### RNA-Interferenz in adhärenten Zellen, vermittelt durch siRNA-beladene VLP, welche mit PEI-Tf komplexiert sind

Die mit siRNA-Molekülen beladenen VLP wurden zu dem Kulturmedium verschiedener humaner Zelllinien zugesetzt. Mehrere Experimente wurden an HeLa SS6-Zellen durchgeführt. 24 Stunden vor der Behandlung mit den VLP wurden die Zellen ausplattiert. Die Untersuchungen erfolgten 24 oder 48 Stunden nach dieser Behandlung.

### HeLa-Zellen

### 1. Dosisabhängigkeit und funktionelle RNA-Interferenz durch PEI-Tf-VLPvermitteltes Einbringen von siRNA-Molekülen.

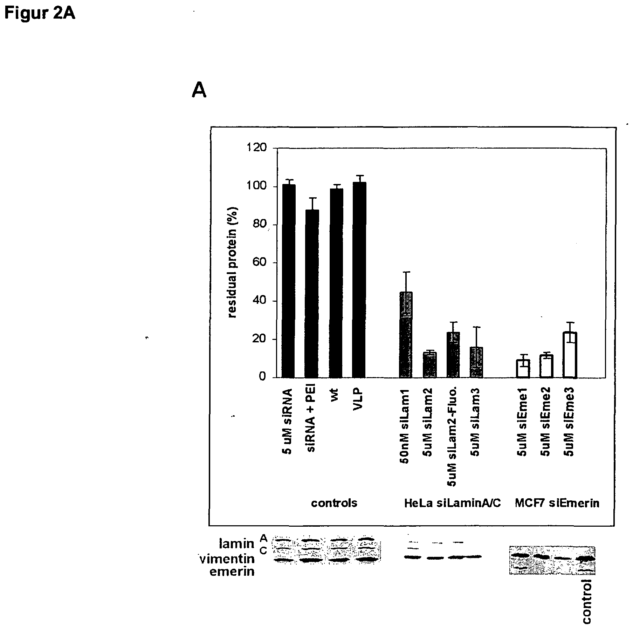

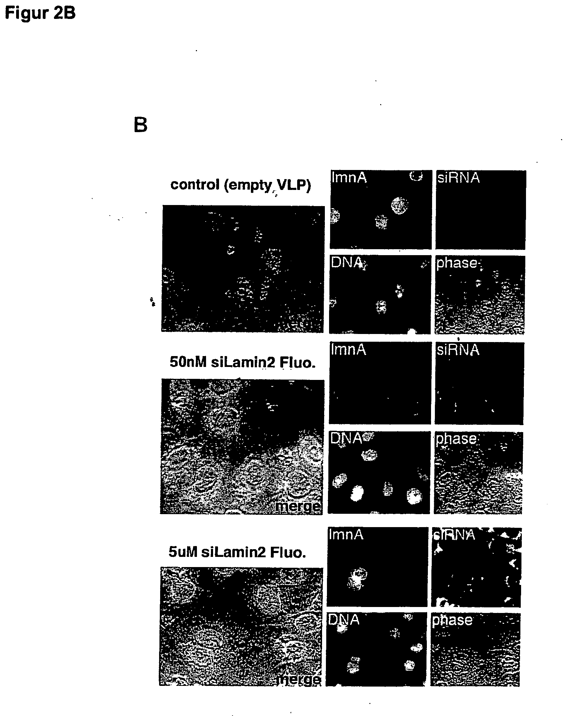

HeLa-Zellen wurden mit einer Dichte von etwa 75.000 Zellen/well in 24-Well-Platten ausplattiert und nach 24 Stunden mit 1 oder 5 µg VLP behandelt. Um die benötigte Menge an siRNA-Molekülen für eine funktionelle und wirksame RNA-Interferenz zu bestimmen, wurden die VLP mit 50 nM oder 3 µM siRNA in Reassemblierungspuffer beladen. Leere VLP, VLP, welche mit nicht verwandten siRNA-Molekülen beladen wurden und siRNA-Moleküle, welche nur mit PEI-Tf komplexiert waren, dienten als Kontrollen. PEI-Tf wurde in einem Verhältnis von 1 zu 5 (1 µg Pei-Tf:5 µg siRNA-VLP) zu den beladenen und reassemblierten VLP zugesetzt, um die Aufnahme in die Zellen mittels des Transferrinrezeptors zu gestatten. Für eine effiziente RNA-Interferenz in etwa 100.000 Zellen je Well einer 24-WellPlatte war es notwendig, diese mit 5 µg VLP zu behandeln, wobei die VLP mit 3 µM siRNA beladen wurden (Fig. 2). Die Transduktion von siRNA mit VLP, welche mit 50 nM siRNA beladen wurden, führte nur zu einer geringen Abnahme des Zielproteins Lamin A(/C). Die Aufnahme von siRNA wurde mittels Transduktion fluoreszenzmarkierter siRNA siLamA-F bestätigt und die Auswirkungen der Herabregulation wurden auf Proteinebene mittels quantitativer Western Blot-Analyse und indirekter Immunfluoreszenzmikroskopie untersucht. Die Transduktion von Zellen mit leeren Pei-Tf-VLP oder mit VLP, welche eine nicht verwandte siRNA enthielten, führte nicht zu einer Abnahme des Lamin A(/C)-Proteingehalts. PEI-Tf komplexierte siRNA ohne VLP verursachte eine schwache Inhibierung der Lamin A(/C)-Expression, wobei die Herabregulation jedoch auf 15-20 % beschränkt war, wohingegen siLamA-PEI-Tf-VLP eine nahezu vollständige Herabregulation ("knockdown") des Zielproteins verursachten, wenn sie in der geeigneten Konzentration verwendet wurden (Fig. HeLaA). Es wurden drei verschiedene siRNA-Moleküle, welche gegen Lamin A/C gerichtet waren, an HeLa-Zellen untersucht, von denen eine fluoreszenzmarkiert war, um die Aufnahme der VLPs in die Zellen und die Aufnahme in das Zytoplasma zu visualisieren. Alle siRNA-Moleküle führten zu einer wirksamen Herabregulation der Zielproteinexpression, wenn diese den Zellen in Form von PEI-Tf-VLP verabreicht wurden.

### 2. Nicht-klassische 27mer dsRNA-Moleküle wurden erfolgreich für eine Herabregulation verwendet

Weiterhin wurde eine nicht-klassische siRNA, d.h. siL27AC, welche aus einer 5'-phosphorylierten dsRNA mit einer Länge von 27 Nukleotiden ohne 3'-Überhänge bestand, in HeLa-Zellen transduziert, in deren Folge die Expression des Lamin A/C-Proteins wirksam herabreguliert wurde. Somit ist es mit der erfindungsgemäßen Zusammensetzung auch möglich, Substrate für die Endonuklease Dicer durch Beladen der VLP mit einer 27mer dsRNA in das Zytoplasma einer Zielzelle einzubringen, um sie für die RNA-Interferenzmaschinerie der Zelle zugänglich zu machen.

### 3. PEI-TF VLP wurden zum Einbringen aktiver siRNA-Moleküle in Brustkrebszellen verwendet

Ein zweiter Satz aus drei unterschiedlichen siRNA-Molekülen, welche gegen das humane Emerin-Gen gerichtet waren, wurde wie oben beschrieben in humane Brustkrebs-MCF-7-Zellen transduziert. Eine quantitative Western Blot-Analyse zeigte, dass alle drei in diesen Experimenten verwendeten siRNA-Spezies erfolgreich eine Herabregulation des Zielgens bewirkten (Fig. 2A). Diese Ergebnisse unterstreichen die breite Anwendbarkeit der VLP im Hinblick auf einen effizienten Transfer von siRNA-Molekülen, die gegen alternative Zielgene in verschiedenen Zelltypen gerichtet sind.

Weitere Zelllinien und Informationen können Tabelle 3 entnommen werden, die eine Zusammenfassung zu Nukleinsäuretransferexperimenten mittels VLP in verschiedene Zelllinien widergibt.

### RNA-Interferenz in nicht-adhärenten Zellen, vermittelt durch siRNA beladene VLPs, welche mit PEI-Tf komplexiert sind

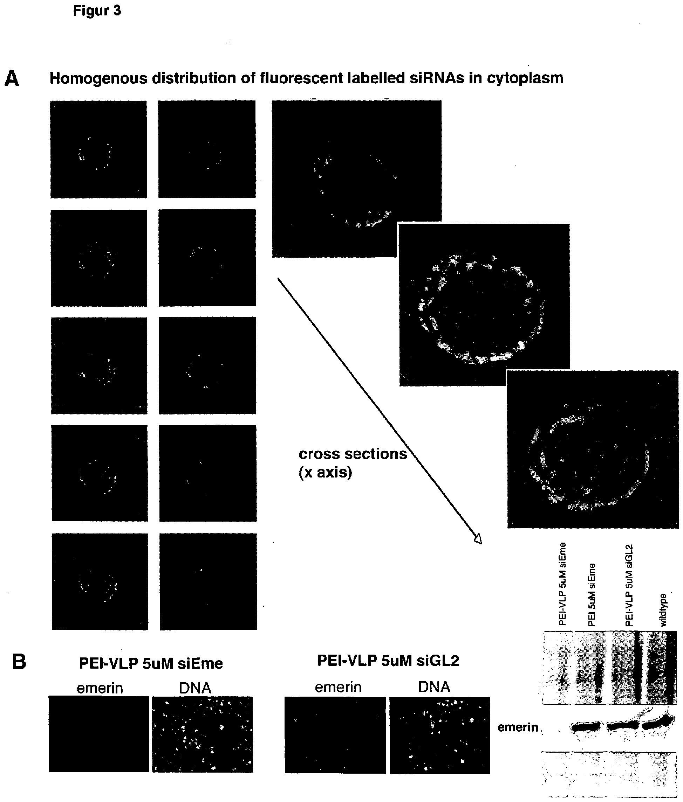

Humane SupT1 T-Zellen wurden in Suspension kultiviert und 24 Stunden von der Behandlung mit VLP ausplattiert. Um den zellulären Energiemetabolismus zu induzieren und die Transferrinrezeptordichte auf der Oberfläche der Zellen zu erhöhen, wurde das Kulturmedium mit 15 nM Desferrioxamin versetzt. Unmittelbar vor der Behandlung mit VLP wurde das Medium durch Standardkulturmedium ersetzt und die VLP wurden in den oben angegebenen Mengen zugesetzt. SupT1-Zellen wurden mit VLP transduziert, welche gegen endogenes humanes Emerin gerichtete siRNA-Moleküle, eine nicht verwandte siRNA (GL2) oder keine siRNA (Fig. 3) enthielten. Dieselben VLP wurden als eine Kontrolle mit und ohne PEI-Transferrin verwendet. Als zusätzliche Kontrolle wurden Zellen PEI-Tf komplexierten siRNA-Molekülen ausgesetzt, wobei diese RNA nicht in VLP inkorporiert war. Um die Aufnahme der siRNA-Moleküle in das Zytoplasma der Zellen zu zeigen, wurde eine fluoreszenzmarkierte siRNA verwendet. Mittels konfokaler Mikroskopie wurde die homogene Verteilung der fluoreszierenden siRNA im Zytoplasma von SupT1-Zellen gezeigt. Schnitte entlang der X-Achse einer solchen Zelle sind in Fig. 3A gezeigt. Die wirksame Herabregulation der Zielgenexpression mittels VLP eingebrachter siRNA wurde mittels indirekter Immunfluoreszenzmikroskopie (Fig. 3B) und Western Blot-Analyse (Fig. 3C) bestätigt. Die Ergebnisse zeigen eine sehr effiziente Herabregulation der Emerin-Genexpression in nahezu 100 % der behandelten Zellen, während die Behandlung mit nicht verwandter siRNA den Gehalt an Emerin nicht beeinflusst. Die Western Blot-Daten zeigen, dass eine hinreichende Herabregulation des Emerins nur erreicht werden konnte, wenn die Zellen mit siEme-beladenen PEI-Tf komplexierten VLP behandelt wurden, während eine PEI-Tf-Komplexierung allein nicht zu einer messbaren Herabregulation des Proteins führt.

Ebenso war eine VLP-vermittelte siRNA-Aufnahme erfolgreich, wenn HeLa S3-Zellen verwendet wurden. Diese Zellen sind auf das Wachstum in Suspension angepasst (Daten nicht gezeigt).

**Tabelle 3: Zusammenfassung der Experimente, Zelllinien, siRNA-Moleküle, RNA- und DNA-Konstrukte und experimentelle Ergebnisse.**

| *Experiment* | *Zelllinie* | *SiRNA*/*Zielgen* | *Aufnahme*/*knoc kdown* | *Anmerkung*/*Erklär ung* |
|---|---|---|---|---|
| **VLP-vermittelte zelluläre Aufnahme fluoreszierend ender siRNA-Moleküle in adhärenten Zellen** | HeLa SS6 (menschlich) | siEg5-F | Ja | siRNA-Moleküle wurden erfolgreich dosisabhängig in das Zytoplasma in >90 % der Zellen eingebracht, was durch Immunfluoreszenz mikroskopie gezeigt wurde; eine Behandlung mit PEI allein ist nicht hinreichend für ein wirksames Einbringen von RNAi induzierenden Molekülen in eine Zielzelle. |
| | | siLamA-F | | |
| | Cos-7 (Afrikanische | siEg5-F | Ja | |
| | gelbgrüne Meerkatze) | siLamA-F | | |
| | Chondrozyten (menschlich) | siEg5-F | Ja | |
| | HeLa (menschlich) | Lamin A/C (drei verschiedene siRNA-Moleküle, eine chemisch modifiziert, d.h. siLamA1, siLamA2, siLamA-F) | Hocheffizienter knockdown (>90 %) Hocheffizienter knockdown | Die mittels VLP eingebrachten siRNA-Moleküle bewirken eine Herabregulation der Expression eines Zielgens, die mit einer klassischen siRNA-Transfektion (z.B. durch kationische Liposomen oder Elektroporation) vergleichbar oder gegenüber dieser besser ist. Ein knockdown wurde mittels IIF und Western Blot bestätigt. Im Fall der Herabregulation von Eg5 zeigten sich die erwarteten Sekundäreffekte, d.h. ein mitotischer Arrest konnte in allen Zellen beobachtet werden. |
| **RNAi mittels VLP eingebrachter siRNA- Moleküle** | | siEg5-F | | |
| | Cos-7 (Afrikanische gelbgrüne Meerkatze) | siEg5-F | Hocheffizientes Einbringen und hocheffizienter knockdown | |
| | Chondrozyten (menschlich) | Lamin A/C (zwei verschiedene siRNA-Moleküle, siLamA2, siLamA-F) | Knockdown nur in wenigen Zellen | Die Zellen proliferierten nicht, was besagt, dass der experimentelle Zeitrahmen für eine Herabregulation des Proteins Lamin A nicht ausreichend war, da dieses Protein eine lange Halbwertszeit aufweist (ein Abbau erfordert einen Mitoseverlauf). |
| | MCF-7 (Menschliches Mammakarzinom | siEme1, siEme2, siEme3, siLamA1 | Effizienter knockdown, dokumentiert durch Western Blot | |
| | 293T (menschliche Embryoniere) | siEme1 | Effizienter knockdown, dokumentiert durch Western Blot | |
| **RNAi mittels 27mer dsRNA-Molekülen** | HeLa SS6 (menschlich) | siL27A | Effizienter knockdown (nicht gezeigt in IIF und Western Blot) | 27mere stumpfendiger dsRNA-Moleküle wurden erfolgreich transduziert und durch Dicer prozessiert. |
| **Zellen in Suspension** | SupT1 | siLamA-F, siEme1 | Aufnahme (nahe 100 %) und funktionelle RNA-Interferenz gegen Emerin (>85 % Herabregulation) wurden sowohl in 1F als auch im Westem Blot gezeigt | SupT1-Zellen erfordern eine Vorbehandlung mit Desferrioxaminase, um die Transferrinrezeptor dichte zu erhöhen. |
| | HeLa S3, | siLamA-F, | Aufnahme wurde gezeigt | |
| **Lineares polll-eGFP-Expressionskonstrukt** | SupT1, Glioma SW103 | polll-eGFP lineare DNA (2,4kb) | Einbringen in nahezu alle Zellen, eGFP wurde entsprechend in beiden Zelllinien exprimiert | Zeigt die Möglichkeit, linearisierte DNA für einen Genaustausch oder für eine miRNA-Expression zu transduzieren |
| **Verpackungseffinzienz** | *In vitro* Studie | Mit siRNA beladene VLP | >80 % der VLPs waren mit siRNA-Molekülen nach der Reassemblierung beladen | |
| **Zytotoxische Studien** | Alle Zelllinien/Tiere | Alle siRNA, insbesondere für eine Therapie | Es wurden keine Auswirkungen auf die Zellvitalität beobachtet | |

### Beispiel 2

Es wurde ein modifiziertes Protein VP1-Mut2 hergestellt, welches gegenüber der Wildtypsequenz eine Modifikation im aminoterminalen Bereich aufweist. Durch diese Modifikation wurde die Aminosäuresequenz eines heterologen Kernlokalisierungssignals basierend auf der Sequenz der Viren SV40 oder BKV eingeführt. Die Sequenz des heterologen Kernlokalisierungssignals ist in Fig. 4A gezeigt.

Das chimäre Protein VP1-Mut2 wurde auf die Fähigkeit getestet, intakte VLPs zu bilden, was durch elektronenmikroskopische Aufnahmen belegt wurde. Des Weiteren wurden verschiedene Zelltypen mit DNA-beladenen VP1-Mut2 VLPs transduziert und die chimäre Form zeigte dabei mindestens gleichwertige (COS-7 Nierenzellen) oder um bis auf das fünffache erhöhte (SVG Gliazellen) Transduktionseffizienzen. Die Ergebnisse sind in Fig. 4B und 4C gezeigt.

### Beispiel 3

Durch heterobifunktionale Linker können Liganden direkt an das VP1-Protein gebunden werden. Eine bevorzugte Ausführungsform für ein solches Kopplungsverfahren ist in Figur 5 dargestellt.

### Beschreibung der Figuren

**Figur 1****:** Effizientes und funktionelles Einbringen von siRNA-Molekülen und DNA in Glioma SW103-Zellen
   A) Zellen wurden mit siLamA1 oder siGL2 siRNA-beladenen VLP behandelt und 44 Stunden nach der VLP-Behandlung mittels indirekter Immunfluoreszenz (IIF) untersucht. Das linke Feld zeigt eine Gruppe von Zellen, die mit siLamA1-VLP behandelt wurden, wobei die Zellen eine nahezu vollständige Herabregulation des Targetproteins (oben) zeigen. Die mit den nicht verwandten Kontroll-siGL2-VLP behandelten Kontrollzellen zeigen einen normalen Lamin A/C-Gehalt, wobei Lamin A/C in den Zellkernen der Zellen vorliegt. Die DNA wurde mit Hoechst 33342 angefärbt.
   B) Mit einer Western Blot-Analyse wurden die Beobachtungen aus der direkten Immunfluoreszenzmikroskopie bestätigt. Zellextrakte von unbehandelten Zellen oder von Zellen, die mit siLamA1-VLP oder siGL2-VLP behandelt wurden, wurden 44 Stunden nach der Behandlung geerntet und mit einem anti-Lamin A/C-Antikörper angefärbt. Der Lamin A/C-Gehalt war in beiden Kontrollspuren gleich, wobei der Anteil an Lamin A/C in siRNA-VLP-behandelten Populationen deutlich rediziert war. Um einen gleichmäßigen Proteinauftrag zu bestätigen, wurden die Blots parallel hinsichtlich Vimentin analysiert.
   C) Zusätzlich zu den siRNA-Molekülen wurde ein linearisiertes Expressionskonstrukt kodierend für eGFP in VLP verpackt und in Gliomazellen transduziert. Nahezu alle Zellen zeigten 24 Stunden nach dieser Behandlung eine positive GFP-Expression.
**Figur 2****:** Qualitative und quantitative Dokumentation des erfolgreichen Einbringens von siRNA mittels modifizierter VLP.
   A) Das Diagramm zeigt die Daten einer quantitativen Western Blot-Analyse von drei einzeln durchgeführten Experimenten, bei denen siRNA in HeLa SS6-Zellen und MCF7-Zellen mittels PEI-Tf-VLP eingebracht wurde. Die Behandlung der Zellen mit PEI-Tf und siRNA, siRNA allein, leeren PEI-Tf-VLP und PEI-Tf-VLP, welche mit nicht verwandten siRNA-Molekülen beladen waren, dienten als individuelle Negativkontrollen. Einzig die Behandlung von PEI-Tf und siLamA verursachte eine schwache (15-20 %) Reduktion des Zielproteingehalts, wohingegen bereits siRNA, welche bei einem Gehalt von 50 nM in VLPs verpackt wurde, effizienter war. Die Beladung der VLP mit 5 µM siRNA und die nachfolgende Komplexierung mit PEI-Tf führte zu einer nahezu vollständigen Herabregulation von Lamin A/C. Andere, gegen Lamin A/C gerichtete siRNA-Moleküle, von denen eine am 5'-Ende des Sinnstranges mit und ohne Fluorescein konjugiert war, zeigten hinsichtlich der Herabregulation in HeLa-Zellen vergleichbare Wirksamkeiten. Ein zweiter Satz von siRNA-Molekülen, die gegen humanes Emerin gerichtet waren, wurde an MCF-7-Zellen untersucht. Wie durch quantitative Western Blot-Analyse gezeigt, wurde die siRNA in allen drei Fällen erfolgreich in die Zellen eingebracht. Das untere Feld zeigt repräsentative Blots nach ECL-Entwicklung. Vimentin wurde in allen Fällen als Ladekontrolle verwendet.
   B) Ein fluoreszenzmikroskopisches Bild von HeLa-Zellen, welche mit siLamA-F-PEI-Tf-VLPs behandelt wurden, zeigt die Aufnahme von siRNA-Molekülen (grün) und die dosisabhängige Herabregulation von Lamin A/C (rot). Verglichen mit der höheren Beladungskonzentration (unteres Feld) sind die Auswirkungen auf die Herabregulation eines Zielgens schwächer, wenn siRNA bei einer Konzentration von 50 nM (mittleres Feld) in VLP inkorporiert wird. In den Kontrollzellen ist eine Reduktion des Lamin A/C-Gehalts nicht zu beobachten, wenn diese mit VLP behandelt wurden, die nicht verwandte siRNA-Moleküle enthalten (oberes Feld).
**Figur 3****:** Transiente RNA-Interferenz in humanen T-Zellen.
   Humane SupT1-Zellen wurden 24 Stunden vor der Behandlung mit siRNA-beladenen PEI-Tf-VLP mit Desferrioxaminase stimuliert.
   A) Eine konfokale Fluoreszenzmikroskopie einer einzelnen supT1-Zelle 24 Stunden nach der Behandlung mit VLP zeigt eine homogene Verteilung fluoreszierender siRNA-Moleküle (siLamA-F) im Zytoplasma. Schnitte entlang der Z-Achse zeigen, dass die Anwesenheit der siRNA hauptsächlich auf das Zytoplasma beschränkt ist, und kaum ein Fluoreszenzsignal an der Peripherie oder im Zellkern zu beobachten ist.
   B) Anti-Emerin-siRNA-siEme1 zeigt nach dem Einbringen in die Zelle deutlich, dass diese siRNA für die RNA-Interferenzmaschinerie der Zelle zugänglich ist. Eine indirekte Immunfluoreszenzmikroskopie mit anti-Emerin-Antikörpern zeigte eine starke Herabregulation von Emerin in der siEme-PEI-Tf-VLP-behandelten Population, während der Emeringehalt in der Kontrollpopulation, welche mit siGL2-PEI-Tf-VLP behandelt wurde, unbeeinflusst blieb.
   C) Die Ergebnisse der Immunfluoreszenzmikroskopie wurden mittels Western Blot-Analyse bestätigt. Lediglich die siEme1-PEI-Tf-VLP-behandelte Population zeigte eine Herabregulation von Emerin, während die Kontrollzellen, welche mit siGL2-PEI-Tf-VLP oder leeren PEI-Tf-VLP behandelt wurden, und die Zellen, die nur mit siEme1 und PEI-Tf behandelt wurde, keine Reduktion des Emeringehalts zeigten. Der Auftrag gleicher Proteinmengen wurde durch Färben der Membran mit Ponceau Rot (Farbstoff ist im Hintergrund gezeigt) bestätigt.
**Figur 4****:**
   Sequenzmodifikationen des VP1-Proteins.
   A)Vergleich von aminoterminalen Sequenzen des Polyoma JC-Virus Wildtyp VP1 und des Chimären VP1-Proteins (VP-Mut2), welches aufgrund einer Aminosäureinsertion ein zweiteiliges Kernlokalisierungssignal enthält. Die aus den Viren SV40 und BKV stammenden Sequenzen, auf deren Basis die Modifikationen vorgenommen wurden, sind als Vergleich gezeigt.
   B) Das chimäre VP1-Protein Mut2 zeigte in elektronenmikroskopischen Aufnahmen gleiche De- und Reassemblierungseigenschaften wie das Wildtyp VP1-Protein.
   C) Das chimäre VP1-Protein Mut2 zeigte eine höhere Transduktionseffizienz in SVG-Gliazellen wie das Wildtyp-Protein. Die relative Luciferase-Aktivität wurde nach Transduktion eines Luciferase-Expressionskonstrukts bestimmt.
**Figur 5****:**
   Durch Kopplung von Liganden an Lysinreste von VP1 ist ein Retargeting möglich. Ein Beispiel für ein heterobifunktionales Linkermolekül, das Aminoan Sulfhydrylgruppen koppeln kann, ist oben gezeigt. Mit Hilfe dieses Linkermoleküls (oder analoger Linkermoleküle) können Lysinreste auf einem ersten Protein (z.B. VP1) und Cysteinreste auf einem zweiten Protein (z.B. Ligand) miteinander verbunden werden. Im mittleren Teil der Figur ist die berechnete Struktur des JCV-VP1 Proteins und schematisch das an einen zugänglichen Lysinrest gekoppelte Linkermolekül gezeigt. Ganz unten ist das JCV-VP1-Protein in einer Ansicht von oben (d.h. vom Äußeren des Kapsids) gezeigt. Die beiden zur Kopplung von Liganden geeigneten Lysinreste K60 und K164 sind angegeben. Diese Lysinreste können zur Anlagerung eines heterobifunktionalen Linkermoleküls wie oben gezeigt, eingesetzt werden.

## Patentansprüche

1. Modifiziertes JCV-VP1 Kapsidprotein, das ein heterologes Kernlokalisierungssignal im Bereich der 25 N-terminalen Aminosäuren, enthält, wobei das heterologe Kernlokalisierungssignal die Aminosäuresequenz CPGAAPX₁X₂P (SEQ ID NO: 7) enthält, wobei X₁ und X₂ beliebige Aminosäuren und vorzugsweise jeweils K bedeuten.

2. Modifiziertes JCV-VP1 Kapsidprotein nach Anspruch 1, wobei das heterologe Kernlokalisierungssignal die Kernlokalisierungssignale von SV40 (SEQ ID NO: 5) oder BKV (SEQ ID NO: 6) umfasst.

3. Nukleinsäure die für ein modifiziertes Kapsidprotein nach einem der Ansprüche 1 oder 2 kodiert.

4. Zusammensetzung umfassend ein virusähnliches Partikel (VLP), welches mindestens ein virales Kapsidprotein nach einem der Ansprüche 1 oder 2 enthält.

5. Zusammensetzung nach Anspruch 4, **dadurch gekennzeichnet, dass** im Inneren des VLP mindestens ein RNA-Interferenz (RNAi-) induzierendes Molekül enthalten ist.

6. Zusammensetzung nach Anspruch 5, wobei das RNAi-induzierende Molekül eine RNA und/oder ein RNA-Analogon ist, oder worin das RNAi-induzierende Molekül eine DNA und oder ein DNA-Analogon ist, welche für siRNA, miRNA, dsRNA, shRNA oder deren Vorläufer kodiert, wobei die DNA gegebenenfalls weiterhin Regulatorelemente enthält, die eine Expression in einer Zielzelle gestatten.

7. Zusammensetzung nach einem der Ansprüche 4 bis 6, **dadurch gekennzeichnet, dass** im Inneren des VLP mindestens eine immunstimulatorische Nukleinsäure gegebenenfalls in Kombination mit Polypeptid- oder Peptid-Immunogen, ein Aptamer oder ein siDNA-Molekül enthalten ist.

8. Zusammensetzung nach einem der Ansprüche 5 bis 7 zur Verwendung zum Einbringen RNAi-induzierender Molekülen in eine Zielzelle, bevorzugt in eine eukaryontische Zelle.

9. Zusammensetzung nach einem der Ansprüche 5 bis 7 zur Verwendung als Arzneimittel.

10. Zusammensetzung nach einem der Ansprüche 6, 7 und Zusammensetzung zur Verwendung gemäß Anspruch 9 zur Verwendung zur Prävention und/oder der Behandlung von Krankheiten oder Krankheitszuständen, die durch Expression einer Nukleinsäure, welche aus einem pathogenen Organismus stammt oder daraus abgeleitet ist, oder durch erhöhte oder unerwünschte Expression eines endogenen Gens verursacht werden.

11. Verwendung einer Zusammensetzung nach einem der Ansprüche 6 und 7 zur in vitro Diagnose von Krankheiten oder Krankheitszuständen, die durch Expression einer Nukleinsäure, welche aus einem pathogenen Organismus stammt oder daraus abgeleitet ist, oder durch erhöhte oder unerwünschte Expression eines endogenen Gens verursacht werden.

12. In vitro Verfahren zur Herstellung einer Zusammensetzung nach einem der Ansprüche 5 oder 6, umfassend das Assemblieren der viralen Kapsidproteine zu VLP in Anwesenheit der RNAi-induzierenden Moleküle.

13. In vitro Verfahren zum Einbringen RNAi-induzierender Moleküle in eine Zielzelle, umfassend die Schritte
(i) Assemblieren der viralen Kapsidproteine umfassend mindestens ein modifiziertes Kapsidprotein nach einem der Ansprüche 1 oder 2 zu VLP in Anwesenheit von RNAi-induzierenden Moleküle, und
(ii) Inkontaktbringen des mit den RNAi-induzierenden Molekülen beladenen VLP aus Schritt (i) mit der Zielzelle unter Bedingungen, bei denen eine Aufnahme der RNAi-induzierenden Moleküle in die Zielzelle erfolgen kann.

14. In vitro Verfahren zur Herabregulation mindestens eines Gens in einer Zielzelle, umfassend die Schritte
(i) Bereitstellen einer Zusammensetzung nach einem der Ansprüche 5 bis 7, und
(ii) Inkontaktbringen der Zusammensetzung aus Schritt (i) mit der Zielzelle unter Bedingungen, bei denen eine Aufnahme der RNAi- induzierenden Moleküle in die Zielzelle erfolgen kann.

## Claims

1. A modified JCV-VP1 capsid protein comprising a heterologous nuclear localization signal located in the 25 N-terminal amino acids thereof, wherein said heterologous nuclear localization signal comprises the amino acid sequence CPGAAPX₁X₂P (SEQ ID NO: 7), wherein X₁ and X₂ can be any amino acid, and preferably are K, respectively.

2. The modified JCV-VP1 capsid protein according to claim 1, wherein said heterologous nuclear localization signal comprises the nuclear localization signal of SV40 (SEQ ID NO: 5) or BKV (SEQ ID NO: 6).

3. A nucleic acid encoding for the modified capsid protein according to any one of claims 1 or 2.

4. A composition comprising a virus-like particle (VLP) containing at least one viral capsid protein according to any one of claims 1 or 2.

5. The composition according to claim 4, **characterized in that** at least one RNA-interference (RNAi) inducing molecule is included in said VLP.

6. The composition according to claim 5, wherein said RNAi-inducing molecule is an RNA and/or an RNA-analogue, or wherein said RNAi-inducing molecule is a DNA and/or a DNA-analogue encoding for siRNA, miRNA, dsRNA, shRNA or a precursor thereof, wherein said DNA optionally further contains regulatory elements allowing the expression in a target cell.

7. The composition according to any one of claims 4 to 6, **characterized in that** at least one immune-stimulatory nucleic acid, optionally in combination with a polypeptide- or peptide-immunogen, an aptamer or an siDNA molecule, is included in the VLP.

8. The composition according to any one of claims 5 to 7 for use in introducing RNAi-inducing molecules into a target cell, preferably into a eukaryotic cell.

9. The composition according to any one of claims 5 to 7 for use as a pharmaceutical composition.

10. The composition according to any one of claims 6, 7, and the composition for use according to claim 9, for use in the prevention and/or treatment of diseases or disease conditions that are caused by the expression of a nucleic acid stemming from or derived from a pathogenic organism, or by an increased or undesired expression of an endogenous gene.

11. Use of the composition according to any one of claims 6 and 7 for the in vitro diagnosis of diseases or disease conditions that are caused by the expression of a nucleic acid stemming from or derived from a pathogenic organism, or by increased or undesired expression of an endogenous gene.

12. In vitro method for preparing a composition according to any one of claims 5 or 6, comprising assembling said viral capsid proteins to VLP in the presence of said RNAi-inducing molecules.

13. In vitro method for introducing RNAi-inducing molecules into a target cell, comprising the steps of
(i) assembling said viral capsid proteins comprising at least one modified capsid protein according to any one of claims 1 or 2 to VLP in the presence of RNAi-inducing molecules, and
(ii) contacting of said VLP loaded with said RNAi-inducing molecules from step (i) with said target cell under conditions in which the RNAi-inducing molecules can be introduced into said target cell.

14. In vitro method for down-regulating at least one gene in a target cell, comprising the steps of
(i) providing a composition according to any one of claims 5 to 7, and
(ii) contacting said composition of step (i) with said target cell under conditions in which the RNAi-inducing molecules can be introduced into said target cell.

## Revendications

1. Protéine de capside JCV-VP1 modifiée, qui comprend un signal de localisation de noyau hétérologue dans le domaine des 25 acides aminés N-terminaux, le signal de localisation de noyau hétérologue comprenant la séquence d'acides aminés CPGAAPX₁X₂P (SÉQ ID N°7), dans laquelle X₁ et X₂ représentent des acides aminés quelconques et de préférence respectivement K.

2. Protéine de capside JCV-VP1 modifiée selon la revendication 1, dans laquelle le signal de localisation de noyau hétérologue comprend les signaux de localisation de noyau de SV40 (SÉQ ID N°5) ou BKV (SÉQ ID N°6).

3. Acide nucléique codant pour une protéine de capside modifiée selon l'une quelconque des revendications 1 ou 2.

4. Composition comprenant une particule de type viral (VLP) qui comprend au moins une protéine de capside virale selon l'une quelconque des revendications 1 ou 2.

5. Composition selon la revendication 4, **caractérisée en ce qu'**au moins une molécule induisant une ARN interférence (ARNi) est présente à l'intérieur de la VLP.

6. Composition selon la revendication 5, dans laquelle la molécule induisant l'ARNi est un ARN et/ou un analogue d'ARN, ou dans laquelle la molécule induisant l'ARNi est un ADN ou un analogue d'ARN codant pour le pARNi, le miARN, l'ARNdb, le shARN ou leurs précurseurs, l'ADN contenant le cas échéant en outre des éléments régulateurs qui permettent une expression dans la cellule cible.

7. Composition selon l'une quelconque des revendications 4 à 6, **caractérisée en ce qu'**au moins un acide nucléique immunostimulant, le cas échéant en combinaison avec un immunogène polypeptidique ou peptidique, un aptamère ou une molécule de pADNi, est présent à l'intérieur de la VLP.

8. Composition selon l'une quelconque des revendications 5 à 7 pour une utilisation pour l'intégration de molécules induisant l'ARNi dans une cellule cible, de préférence dans une cellule eucaryote.

9. Composition selon l'une quelconque des revendications 5 à 7 pour une utilisation en tant que médicament.

10. Composition selon l'une quelconque des revendications 6, 7 et composition pour une utilisation selon la revendication 9 pour l'utilisation dans la prévention et/ou le traitement des pathologies ou des états pathologiques qui sont provoqués par l'expression d'un acide nucléique qui provient d'un organisme pathogène ou en est dérivé, ou par une expression accrue ou indésirable d'un gène endogène.

11. Utilisation d'une composition selon l'une quelconque des revendications 6 et 7 pour le diagnostic in vitro de pathologies ou d'états pathologiques qui sont provoqués par l'expression d'un acide nucléique qui provient d'un organisme pathogène ou en est dérivé, ou par une expression accrue ou indésirable d'un gène endogène.

12. Procédé in vitro pour la préparation d'une composition selon l'une quelconque des revendications 5 ou 6, comprenant l'assemblage de protéines de capside virales en une VLP en présence de molécules induisant l'ARNi.

13. Procédé in vitro d'intégration de molécules induisant l'ARNi dans une cellule cible, comprenant les étapes de
(i) assemblage de protéines de capside virales comprenant au moins une protéine de capside modifiée selon l'une quelconque des revendications 1 ou 2 en une VLP en présence de molécules induisant l'ARNi, et
(ii) mise en contact de la VLP chargée de molécules induisant l'AiRN provenant de l'étape (i) avec la cellule cible dans des conditions où une absorption des molécules induisant l'ARNi dans la cellule cible peut s'effectuer.

14. Procédé in vitro de régulation à la baisse d'au moins un gène dans une cellule cible comprenant les étapes de
(i) mise à disposition d'une composition selon l'une quelconque des revendications 5 à 7, et
(ii) mise en contact de la composition provenant de l'étape (i) avec la cellule cible dans des conditions où une absorption des molécules induisant l'ARNi dans la cellule cible peut s'effectuer.
